# EUROPEAN PATENT APPLICATION

(11) **EP 3 943 503 A1**
(43) Date of publication of application: **26.01.2022**
(21) Application number: 19912419.9
(22) Date of filing: 24.09.2019
(51) Int. Cl.: C07K 14/015, C12N 15/35, C12N 7/01, A61K 48/00, A61P 27/16

(54) **ADENO-ASSOCIATED VIRUS HAVING VARIANT CAPSID PROTEIN AND USE THEREOF**

(30) Priority: 30.01.2019 CN 201910093000; 29.08.2019 CN 201910807643
(71) Applicant: Shanghaitech University, Shanghai 201210 (CN)
(72) Inventor: TAN, Fangzhi, Shanghai 201210 (CN); ZHONG, Guisheng, Shanghai 201210 (CN); CHU, Cenfeng, Shanghai 201210 (CN); QI, Jieyu, Shanghai 201210 (CN)
(74) Representative: Canzler & Bergmeier Patentanwälte Partnerschaft mbB
(86) International application number: PCT/CN2019/107474
(87) International publication number: WO 2020/155655

(57) **Abstract**

Provided are an adeno-associated virus having a variant capsid protein and the use thereof. The variant adeno-associated virus AAV-ie is obtained by inserting an amino acid sequence DGTLAVPFK between N589 and R590 of the wild-type AAV-DJ capsid protein VPI. The variant adeno-associated virus AAV-ie can efficiently infect hair cells and supporting cells, and is greatly improved compared with parents thereof, thereby providing better technical support for scientific research.

SEQUENCE LISTING

<110>  ShanghaiTech University

<120>  ADENO-ASSOCIATED VIRUS HAVING A VARIANT CAPSID PROTEIN, AND USE
       THEREOF

<150>  2019100930003
<151>  2019-01-30

<160>  12

<170>  SIPOSequenceListing 1.0

<210>  1
<211>  9
<212>  PRT
<213>  Artificial Sequence

<400>  1

Asp Gly Thr Leu Ala Val Pro Phe Lys
1               5


<210>  2
<211>  27
<212>  DNA
<213>  Artificial Sequence

<400>  2
gatgggactt tggcggtgcc ttttaag                                          27


<210>  3
<211>  737
<212>  PRT
<213>  Artificial Sequence

<400>  3

Met Ala Ala Asp Gly Tyr Leu Pro Asp Trp Leu Glu Asp Thr Leu Ser
1               5                   10                  15


Glu Gly Ile Arg Gln Trp Trp Lys Leu Lys Pro Gly Pro Pro Pro Pro
            20                  25                  30


Lys Pro Ala Glu Arg His Lys Asp Asp Ser Arg Gly Leu Val Leu Pro
        35                  40                  45


Gly Tyr Lys Tyr Leu Gly Pro Phe Asn Gly Leu Asp Lys Gly Glu Pro
    50                  55                  60


Val Asn Glu Ala Asp Ala Ala Ala Leu Glu His Asp Lys Ala Tyr Asp
65                  70                  75                  80


Arg Gln Leu Asp Ser Gly Asp Asn Pro Tyr Leu Lys Tyr Asn His Ala
                85                  90                  95

Asp Ala Glu Phe Gln Glu Arg Leu Lys Glu Asp Thr Ser Phe Gly Gly
            100                 105                 110

Asn Leu Gly Arg Ala Val Phe Gln Ala Lys Lys Arg Leu Leu Glu Pro
        115                 120                 125

Leu Gly Leu Val Glu Glu Ala Ala Lys Thr Ala Pro Gly Lys Lys Arg
    130                 135                 140

Pro Val Glu His Ser Pro Val Glu Pro Asp Ser Ser Ser Gly Thr Gly
145                 150                 155                 160

Lys Ala Gly Gln Gln Pro Ala Arg Lys Arg Leu Asn Phe Gly Gln Thr
                165                 170                 175

Gly Asp Ala Asp Ser Val Pro Asp Pro Gln Pro Ile Gly Glu Pro Pro
            180                 185                 190

Ala Ala Pro Ser Gly Val Gly Ser Leu Thr Met Ala Ala Gly Gly Gly
        195                 200                 205

Ala Pro Met Ala Asp Asn Asn Glu Gly Ala Asp Gly Val Gly Asn Ser
    210                 215                 220

Ser Gly Asn Trp His Cys Asp Ser Thr Trp Met Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Asn Ser Thr Ser Gly Gly Ser Ser Asn Asp Asn
            260                 265                 270

Ala Tyr Phe Gly Tyr Ser Thr Pro Trp Gly Tyr Phe Asp Phe Asn Arg
        275                 280                 285

Phe His Cys His Phe Ser Pro Arg Asp Trp Gln Arg Leu Ile Asn Asn
    290                 295                 300

Asn Trp Gly Phe Arg Pro Lys Arg Leu Ser Phe Lys Leu Phe Asn Ile
305                 310                 315                 320

Gln Val Lys Glu Val Thr Gln Asn Glu Gly Thr Lys Thr Ile Ala Asn
                325                 330                 335

Asn Leu Thr Ser Thr Ile Gln Val Phe Thr Asp Ser Glu Tyr Gln Leu

            340                 345                 350

Pro Tyr Val Leu Gly Ser Ala His Gln Gly Cys Leu Pro Pro Phe Pro
        355                 360                 365

Ala Asp Val Phe Met Ile Pro Gln Tyr Gly Tyr Leu Thr Leu Asn Asn
    370                 375                 380

Gly Ser Gln Ala Val Gly Arg Ser Ser Phe Tyr Cys Leu Glu Tyr Phe
385                 390                 395                 400

Pro Ser Gln Met Leu Arg Thr Gly Asn Asn Phe Gln Phe Thr Tyr Thr
                405                 410                 415

Phe Glu Asp Val Pro Phe His Ser Ser Tyr Ala His Ser Gln Ser Leu
            420                 425                 430

Asp Arg Leu Met Asn Pro Leu Ile Asp Gln Tyr Leu Tyr Tyr Leu Ser
        435                 440                 445

Arg Thr Gln Thr Thr Gly Gly Thr Thr Asn Thr Gln Thr Leu Gly Phe
    450                 455                 460

Ser Gln Gly Gly Pro Asn Thr Met Ala Asn Gln Ala Lys Asn Trp Leu
465                 470                 475                 480

Pro Gly Pro Cys Tyr Arg Gln Gln Arg Val Ser Lys Thr Ser Ala Asp
                485                 490                 495

Asn Asn Asn Ser Glu Tyr Ser Trp Thr Gly Ala Thr Lys Tyr His Leu
            500                 505                 510

Asn Gly Arg Asp Ser Leu Val Asn Pro Gly Pro Ala Met Ala Ser His
        515                 520                 525

Lys Asp Asp Glu Glu Lys Phe Phe Pro Gln Ser Gly Val Leu Ile Phe
    530                 535                 540

Gly Lys Gln Gly Ser Glu Lys Thr Asn Val Asp Ile Glu Lys Val Met
545                 550                 555                 560

Ile Thr Asp Glu Glu Glu Ile Arg Thr Thr Asn Pro Val Ala Thr Glu
                565                 570                 575

Gln Tyr Gly Ser Val Ser Thr Asn Leu Gln Arg Gly Asn Arg Gln Ala
            580                 585                 590

Ala Thr Ala Asp Val Asn Thr Gln Gly Val Leu Pro Gly Met Val Trp
        595                 600                 605

Gln Asp Arg Asp Val Tyr Leu Gln Gly Pro Ile Trp Ala Lys Ile Pro
    610                 615                 620

His Thr Asp Gly His Phe His Pro Ser Pro Leu Met Gly Gly Phe Gly
625                 630                 635                 640

Leu Lys His Pro Pro Pro Gln Ile Leu Ile Lys Asn Thr Pro Val Pro
                645                 650                 655

Ala Asp Pro Pro Thr Thr Phe Asn Gln Ser Lys Leu Asn Ser Phe Ile
            660                 665                 670

Thr Gln Tyr Ser Thr Gly Gln Val Ser Val Glu Ile Glu Trp Glu Leu
        675                 680                 685

Gln Lys Glu Asn Ser Lys Arg Trp Asn Pro Glu Ile Gln Tyr Thr Ser
    690                 695                 700

Asn Tyr Tyr Lys Ser Thr Ser Val Asp Phe Ala Val Asn Thr Glu Gly
705                 710                 715                 720

Val Tyr Ser Glu Pro Arg Pro Ile Gly Thr Arg Tyr Leu Thr Arg Asn
                725                 730                 735

Leu

<210>  4
<211>  746
<212>  PRT
<213>  Artificial Sequence

<400>  4

Met Ala Ala Asp Gly Tyr Leu Pro Asp Trp Leu Glu Asp Thr Leu Ser
1               5                   10                  15

Glu Gly Ile Arg Gln Trp Trp Lys Leu Lys Pro Gly Pro Pro Pro Pro
            20                  25                  30

Lys Pro Ala Glu Arg His Lys Asp Asp Ser Arg Gly Leu Val Leu Pro
        35                  40                  45

Gly Tyr Lys Tyr Leu Gly Pro Phe Asn Gly Leu Asp Lys Gly Glu Pro
    50                  55                  60

Val Asn Glu Ala Asp Ala Ala Ala Leu Glu His Asp Lys Ala Tyr Asp
65                  70                  75                  80

Arg Gln Leu Asp Ser Gly Asp Asn Pro Tyr Leu Lys Tyr Asn His Ala
                85                  90                  95

Asp Ala Glu Phe Gln Glu Arg Leu Lys Glu Asp Thr Ser Phe Gly Gly
            100                 105                 110

Asn Leu Gly Arg Ala Val Phe Gln Ala Lys Lys Arg Leu Leu Glu Pro
        115                 120                 125

Leu Gly Leu Val Glu Glu Ala Ala Lys Thr Ala Pro Gly Lys Lys Arg
    130                 135                 140

Pro Val Glu His Ser Pro Val Glu Pro Asp Ser Ser Ser Gly Thr Gly
145                 150                 155                 160

Lys Ala Gly Gln Gln Pro Ala Arg Lys Arg Leu Asn Phe Gly Gln Thr
                165                 170                 175

Gly Asp Ala Asp Ser Val Pro Asp Pro Gln Pro Ile Gly Glu Pro Pro
            180                 185                 190

Ala Ala Pro Ser Gly Val Gly Ser Leu Thr Met Ala Ala Gly Gly Gly
        195                 200                 205

Ala Pro Met Ala Asp Asn Asn Glu Gly Ala Asp Gly Val Gly Asn Ser
    210                 215                 220

Ser Gly Asn Trp His Cys Asp Ser Thr Trp Met Gly Asp Arg Val Ile
225                 230                 235                 240

Thr Thr Ser Thr Arg Thr Trp Ala Leu Pro Thr Tyr Asn Asn His Leu
                245                 250                 255

Tyr Lys Gln Ile Ser Asn Ser Thr Ser Gly Gly Ser Ser Asn Asp Asn
            260                 265                 270

Ala Tyr Phe Gly Tyr Ser Thr Pro Trp Gly Tyr Phe Asp Phe Asn Arg
        275                 280                 285

Phe His Cys His Phe Ser Pro Arg Asp Trp Gln Arg Leu Ile Asn Asn
    290                 295                 300

Asn Trp Gly Phe Arg Pro Lys Arg Leu Ser Phe Lys Leu Phe Asn Ile
305                 310                 315                 320

Gln Val Lys Glu Val Thr Gln Asn Glu Gly Thr Lys Thr Ile Ala Asn
                325                 330                 335

Asn Leu Thr Ser Thr Ile Gln Val Phe Thr Asp Ser Glu Tyr Gln Leu
            340                 345                 350

Pro Tyr Val Leu Gly Ser Ala His Gln Gly Cys Leu Pro Pro Phe Pro
        355                 360                 365

Ala Asp Val Phe Met Ile Pro Gln Tyr Gly Tyr Leu Thr Leu Asn Asn
    370                 375                 380

Gly Ser Gln Ala Val Gly Arg Ser Ser Phe Tyr Cys Leu Glu Tyr Phe
385                 390                 395                 400

Pro Ser Gln Met Leu Arg Thr Gly Asn Asn Phe Gln Phe Thr Tyr Thr
                405                 410                 415

Phe Glu Asp Val Pro Phe His Ser Ser Tyr Ala His Ser Gln Ser Leu
            420                 425                 430

Asp Arg Leu Met Asn Pro Leu Ile Asp Gln Tyr Leu Tyr Tyr Leu Ser
        435                 440                 445

Arg Thr Gln Thr Thr Gly Gly Thr Thr Asn Thr Gln Thr Leu Gly Phe
    450                 455                 460

Ser Gln Gly Gly Pro Asn Thr Met Ala Asn Gln Ala Lys Asn Trp Leu
465                 470                 475                 480

Pro Gly Pro Cys Tyr Arg Gln Gln Arg Val Ser Lys Thr Ser Ala Asp
                485                 490                 495

Asn Asn Asn Ser Glu Tyr Ser Trp Thr Gly Ala Thr Lys Tyr His Leu
            500                 505                 510

Asn Gly Arg Asp Ser Leu Val Asn Pro Gly Pro Ala Met Ala Ser His
        515                 520                 525

Lys Asp Asp Glu Glu Lys Phe Phe Pro Gln Ser Gly Val Leu Ile Phe
    530                 535                 540

Gly Lys Gln Gly Ser Glu Lys Thr Asn Val Asp Ile Glu Lys Val Met
545                 550                 555                 560

Ile Thr Asp Glu Glu Glu Ile Arg Thr Thr Asn Pro Val Ala Thr Glu
                565                 570                 575

Gln Tyr Gly Ser Val Ser Thr Asn Leu Gln Arg Gly Asn Asp Gly Thr
            580                 585                 590

Leu Ala Val Pro Phe Lys Arg Gln Ala Ala Thr Ala Asp Val Asn Thr
        595                 600                 605

Gln Gly Val Leu Pro Gly Met Val Trp Gln Asp Arg Asp Val Tyr Leu
    610                 615                 620

Gln Gly Pro Ile Trp Ala Lys Ile Pro His Thr Asp Gly His Phe His
625                 630                 635                 640

Pro Ser Pro Leu Met Gly Gly Phe Gly Leu Lys His Pro Pro Pro Gln
                645                 650                 655

Ile Leu Ile Lys Asn Thr Pro Val Pro Ala Asp Pro Pro Thr Thr Phe
            660                 665                 670

Asn Gln Ser Lys Leu Asn Ser Phe Ile Thr Gln Tyr Ser Thr Gly Gln
        675                 680                 685

Val Ser Val Glu Ile Glu Trp Glu Leu Gln Lys Glu Asn Ser Lys Arg
    690                 695                 700

Trp Asn Pro Glu Ile Gln Tyr Thr Ser Asn Tyr Tyr Lys Ser Thr Ser
705                 710                 715                 720

Val Asp Phe Ala Val Asn Thr Glu Gly Val Tyr Ser Glu Pro Arg Pro
                725                 730                 735

Ile Gly Thr Arg Tyr Leu Thr Arg Asn Leu
            740                 745

<210>  5
<211>  2241
<212>  DNA
<213>  Artificial Sequence

<400>  5
atggctgccg atggttatct tccagattgg ctcgaggaca ctctctctga aggaataaga      60

cagtggtgga agctcaaacc tggcccacca ccaccaaagc ccgcagagcg gcataaggac     120

gacagcaggg gtcttgtgct tcctgggtac aagtacctcg gacccttcaa cggactcgac     180

aagggagagc cggtcaacga ggcagacgcc gcggccctcg agcacgacaa agcctacgac     240

cggcagctcg acagcggaga caacccgtac ctcaagtaca accacgccga cgccgagttc     300

caggagcggc tcaaagaaga tacgtctttt gggggcaacc tcgggcgagc agtcttccag     360

gccaaaaaga ggcttcttga acctcttggt ctggttgagg aagcggctaa gacggctcct 420

ggaaagaaga ggcctgtaga gcactctcct gtggagccag actcctcctc gggaaccgga 480

aaggcgggcc agcagcctgc aagaaaaaga ttgaattttg gtcagactgg agacgcagac 540

tcagtcccag accctcaacc aatcggagaa cctcccgcag ccccctcagg tgtgggatct 600

cttacaatgg ctgcaggcgg tggcgcacca atggcagaca ataacgaggg cgccgacgga 660

gtgggtaatt cctcgggaaa ttggcattgc gattccacat ggatgggcga cagagtcatc 720

accaccagca cccgaacctg ggccctgccc acctacaaca accacctcta caagcaaatc 780

tccaacagca catctggagg atcttcaaat gacaacgcct acttcggcta cagcaccccc 840

tgggggtatt ttgactttaa cagattccac tgccactttt caccacgtga ctggcagcga 900

ctcatcaaca acaactgggg attccggccc aagagactca gcttcaagct cttcaacatc 960

caggtcaagg aggtcacgca gaatgaaggc accaagacca tcgccaataa cctcaccagc 1020

accatccagg tgtttacgga ctcggagtac cagctgccgt acgttctcgg ctctgcccac 1080

cagggctgcc tgcctccgtt cccggcggac gtgttcatga ttccccagta cggctaccta 1140

acactcaaca acggtagtca ggccgtggga cgctcctcct tctactgcct ggaatacttt 1200

ccttcgcaga tgctgagaac cggcaacaac ttccagttta cttacacctt cgaggacgtg 1260

cctttccaca gcagctacgc ccacagccag agcttggacc ggctgatgaa tcctctgatt 1320

gaccagtacc tgtactactt gtctcggact caaacaacag gaggcacgac aaatacgcag 1380

actctgggct tcagccaagg tgggcctaat acaatggcca atcaggcaaa gaactggctg 1440

ccaggaccct gttaccgcca gcagcgagta tcaaagacat ctgcggataa caacaacagt 1500

gaatactcgt ggactggagc taccaagtac cacctcaatg gcagagactc tctggtgaat 1560

ccgggcccgg ccatggcaag ccacaaggac gatgaagaaa agttttttcc tcagagcggg 1620

gttctcatct ttgggaagca aggctcagag aaaacaaatg tggacattga aaaggtcatg 1680

attacagacg aagaggaaat caggacaacc aatcccgtgg ctacggagca gtatggttct 1740

gtatctacca acctccagag aggcaacgat gggactttgg cggtgccttt taagagacaa 1800

gcagctaccg cagatgtcaa cacacaaggc gttcttccag gcatggtctg gcaggacaga 1860

gatgtgtacc ttcaggggcc catctgggca aagattccac acacggacgg acattttcac 1920

ccctctcccc tcatgggtgg attcggactt aaacaccctc cgcctcagat cctgatcaag 1980

aacacgcctg tacctgcgga tcctccgacc accttcaacc agtcaaagct gaactctttc 2040

atcacccagt attctactgg ccaagtcagc gtggagatcg agtgggagct gcagaaggaa 2100

aacagcaagc gctggaaccc cgagatccag tacacctcca actactacaa atctacaagt 2160

gtggactttg ctgttaatac agaaggcgtg tactctgaac cccgccccat tggcacccgt 2220

tacctcaccc gtaatctgta a                                              2241

<210>  6
<211>  7360
<212>  DNA
<213>  Artificial Sequence

<400>  6
gcgcgccgat atcgttaacg ccccgcgccg gccgctctag aactagtgga tcccccggaa      60

gatcagaagt tcctattccg aagttcctat tctctagaaa gtataggaac ttctgatctg     120

cgcagccgcc atgccggggt tttacgagat tgtgattaag gtccccagcg accttgacga     180

gcatctgccc ggcatttctg acagctttgt gaactgggtg gccgagaagg aatgggagtt     240

gccgccagat tctgacatgg atctgaatct gattgagcag gcacccctga ccgtggccga     300

gaagctgcag cgcgactttc tgacggaatg gcgccgtgtg agtaaggccc cggaggccct     360

tttctttgtg caatttgaga agggagagag ctacttccac atgcacgtgc tcgtggaaac     420

caccggggtg aaatccatgg ttttgggacg tttcctgagt cagattcgcg aaaaactgat     480

tcagagaatt taccgcggga tcgagccgac tttgccaaac tggttcgcgg tcacaaagac     540

cagaaatggc gccggaggcg ggaacaaggt ggtggatgag tgctacatcc ccaattactt     600

gctccccaaa acccagcctg agctccagtg ggcgtggact aatatggaac agtatttaag     660

cgcctgtttg aatctcacgg agcgtaaacg gttggtggcg cagcatctga cgcacgtgtc     720

gcagacgcag gagcagaaca aagagaatca gaatcccaat tctgatgcgc cggtgatcag     780

atcaaaaact tcagccaggt acatggagct ggtcgggtgg ctcgtggaca aggggattac     840

ctcggagaag cagtggatcc aggaggacca ggcctcatac atctccttca atgcggcctc     900

caactcgcgg tcccaaatca aggctgcctt ggacaatgcg ggaaagatta tgagcctgac     960

taaaaccgcc cccgactacc tggtgggcca gcagcccgtg gaggacattt ccagcaatcg    1020

gatttataaa attttggaac taaacgggta cgatccccaa tatgcggctt ccgtctttct    1080

gggatgggcc acgaaaaagt tcggcaagag gaacaccatc tggctgtttg ggcctgcaac    1140

taccgggaag accaacatcg cggaggccat agcccacact gtgcccttct acgggtgcgt    1200

aaactggacc aatgagaact ttcccttcaa cgactgtgtc gacaagatgg tgatctggtg    1260

ggaggagggg aagatgaccg ccaaggtcgt ggagtcggcc aaagccattc tcggaggaag    1320

caaggtgcgc gtggaccaga aatgcaagtc ctcggcccag atagacccga ctcccgtgat    1380

cgtcacctcc aacaccaaca tgtgcgccgt gattgacggg aactcaacga ccttcgaaca    1440

ccagcagccg ttgcaagacc ggatgttcaa atttgaactc acccgccgtc tggatcatga    1500

ctttgggaag gtcaccaagc aggaagtcaa agactttttc cggtgggcaa aggatcacgt    1560

ggttgaggtg gagcatgaat tctacgtcaa aaagggtgga gccaagaaaa gacccgcccc    1620

cagtgacgca gatataagtg agcccaaacg ggtgcgcgag tcagttgcgc agccatcgac 1680

gtcagacgcg gaagcttcga tcaactacgc agacaggtac caaaacaaat gttctcgtca 1740

cgtgggcatg aatctgatgc tgtttccctg cagacaatgc gagagaatga atcagaattc 1800

aaatatctgc ttcactcacg gacagaaaga ctgtttagag tgctttcccg tgtcagaatc 1860

tcaacccgtt tctgtcgtca aaaaggcgta tcagaaactg tgctacattc atcatatcat 1920

gggaaaggtg ccagacgctt gcactgcctg cgatctggtc aatgtggatt tggatgactg 1980

catctttgaa caataaatga tttaaatcag gtatggctgc cgatggttat cttccagatt 2040

ggctcgagga cactctctct gaaggaataa gacagtggtg gaagctcaaa cctggcccac 2100

caccaccaaa gcccgcagag cggcataagg acgacagcag gggtcttgtg cttcctgggt 2160

acaagtacct cggacccttc aacggactcg acaagggaga gccggtcaac gaggcagacg 2220

ccgcggccct cgagcacgac aaagcctacg accggcagct cgacagcgga gacaacccgt 2280

acctcaagta caaccacgcc gacgccgagt tccaggagcg gctcaaagaa gatacgtctt 2340

ttgggggcaa cctcggcgga gcagtcttcc aggccaaaaa gaggcttctt gaacctcttg 2400

gtctggttga ggaagcggct aagacggctc ctggaaagaa gaggcctgta gagcactctc 2460

ctgtggagcc agactcctcc tcgggaaccg gaaaggcggg ccagcagcct gcaagaaaaa 2520

gattgaattt tggtcagact ggagacgcag actcagtccc agaccctcaa ccaatcggag 2580

aacctcccgc agccccctca ggtgtgggat ctcttacaat ggctgcaggc ggtggcgcac 2640

caatggcaga caataacgag ggcgccgacg gagtgggtaa ttcctcggga aattggcatt 2700

gcgattccac atggatgggc gacagagtca tcaccaccag cacccgaacc tgggccctgc 2760

ccacctacaa caaccacctc tacaagcaaa tctccaacag cacatctgga ggatcttcaa 2820

atgacaacgc ctacttcggc tacagcaccc cctgggggta ttttgacttt aacagattcc 2880

actgccactt ttcaccacgt gactggcagc gactcatcaa caacaactgg ggattccggc 2940

ccaagagact cagcttcaag ctcttcaaca tccaggtcaa ggaggtcacg cagaatgaag 3000

gcaccaagac catcgccaat aacctcacca gcaccatcca ggtgtttacg gactcggagt 3060

accagctgcc gtacgttctc ggctctgccc accagggctg cctgcctccg ttcccggcgg 3120

acgtgttcat gattccccag tacggctacc taacactcaa caacggtagt caggccgtgg 3180

gacgctcctc cttctactgc ctggaatact ttccttcgca gatgctgaga accggcaaca 3240

acttccagtt tacttacacc ttcgaggacg tgcctttcca cagcagctac gcccacagcc 3300

agagcttgga ccggctgatg aatcctctga ttgaccagta cctgtactac ttgtctcgga 3360

ctcaaacaac aggaggcacg acaaatacgc agactctggg cttcagccaa ggtgggccta 3420

atacaatggc caatcaggca aagaactggc tgccaggacc ctgttaccgc cagcagcgag 3480

tatcaaagac atctgcggat aacaacaaca gtgaatactc gtggactgga gctaccaagt 3540

accacctcaa tggcagagac tctctggtga atccgggccc ggccatggca agccacaagg 3600

acgatgaaga aaagtttttt cctcagagcg gggttctcat ctttgggaag caaggctcag 3660

agaaaacaaa tgtggacatt gaaaaggtca tgattacaga cgaagaggaa atcaggacaa 3720

ccaatcccgt ggctacggag cagtatggtt ctgtatctac caacctccag agaggcaacg 3780

atgggacttt ggcggtgcct tttaagagac aagcagctac cgcagatgtc aacacacaag 3840

gcgttcttcc aggcatggtc tggcaggaca gagatgtgta ccttcagggg cccatctggg 3900

caaagattcc acacacggac ggacattttc acccctctcc cctcatgggt ggattcggac 3960

ttaaacaccc tccgcctcag atcctgatca agaacacgcc tgtacctgcg gatcctccga 4020

ccaccttcaa ccagtcaaag ctgaactctt tcatcaccca gtattctact ggccaagtca 4080

gcgtggagat cgagtgggag ctgcagaagg aaaacagcaa gcgctggaac cccgagatcc 4140

agtacacctc caactactac aaatctacaa gtgtggactt tgctgttaat acagaaggcg 4200

tgtactctga accccgcccc attggcaccc gttacctcac ccgtaatctg taattgcttg 4260

ttaatcaata aaccgtttaa ttcgtttcag ttgaactttg gtctctgcgt atttctttct 4320

tatctagttt ccatggctac gtagataagt agcatggcgg gttaatcatt aactacagcc 4380

cgggcgttta aacagcgggc ggaggggtgg agtcgtgacg tgaattacgt catagggtta 4440

gggaggtcct gtattagagg tcacgtgagt gttttgcgac attttgcgac accatgtggt 4500

ctcgctgggg ggggggcccg gagtgagcac gcagggtctc cattttgaag cgggaggttt 4560

gaacgagcgc tggcgcgctc actggccgtc gttttacaac gtcgtgactg ggaaaaccct 4620

ggcgttaccc aacttaatcg ccttgcagca catccccctt tcgccagctg gcgtaatagc 4680

gaagaggccc gcaccgatcg cccttcccaa cagttgcgca gcctgaatgg cgaatggaaa 4740

ttgtaagcgt taatattttg ttaaaattcg cgttaaattt ttgttaaatc agctcatttt 4800

tttaaccaat aggccgaaat cggcaaaatc ccttataaat caaaagaata gaccgagata 4860

gggttgagtg ttgttccagt ttggaacaag agtccactat taagaacgtg gactccaacg 4920

tcaaagggcg aaaaaccgtc tatcagggcg atggcccact acgtgaacca tcaccctaat 4980

caagtttttt ggggtcgagg tgccgtaaag cactaaatcg gaaccctaaa gggagccccc 5040

gatttagagc ttgacgggga aagccggcga acgtggcgag aaaggaaggg aagaaagcga 5100

aaggagcggg cgctagggcg ctggcaagtg tagcggtcac gctgcgcgta accaccacac 5160

ccgccgcgct taatgcgccg ctacagggcg cgtcaggtgg cacttttcgg ggaaatgtgc 5220

gcggaacccc tatttgttta tttttctaaa tacattcaaa tatgtatccg ctcatgagac 5280

aataaccctg ataaatgctt caataatatt gaaaaaggaa gagtatgagt attcaacatt 5340

tccgtgtcgc ccttattccc ttttttgcgg cattttgcct tcctgttttt gctcacccag 5400

aaacgctggt gaaagtaaaa gatgctgaag atcagttggg tgcacgagtg ggttacatcg 5460

aactggatct caacagcggt aagatccttg agagttttcg ccccgaagaa cgttttccaa 5520

tgatgagcac ttttaaagtt ctgctatgtg gcgcggtatt atcccgtatt gacgccgggc 5580

aagagcaact cggtcgccgc atacactatt ctcagaatga cttggttgag tactcaccag 5640

tcacagaaaa gcatcttacg gatggcatga cagtaagaga attatgcagt gctgccataa 5700

ccatgagtga taacactgcg gccaacttac ttctgacaac gatcggagga ccgaaggagc 5760

taaccgcttt tttgcacaac atgggggatc atgtaactcg ccttgatcgt tgggaaccgg 5820

agctgaatga agccatacca aacgacgagc gtgacaccac gatgcctgta gcaatggcaa 5880

caacgttgcg caaactatta actggcgaac tacttactct agcttcccgg caacaattaa 5940

tagactggat ggaggcggat aaagttgcag gaccacttct gcgctcggcc cttccggctg 6000

gctggtttat tgctgataaa tctggagccg gtgagcgtgg gtctcgcggt atcattgcag 6060

cactggggcc agatggtaag ccctcccgta tcgtagttat ctacacgacg gggagtcagg 6120

caactatgga tgaacgaaat agacagatcg ctgagatagg tgcctcactg attaagcatt 6180

ggtaactgtc agaccaagtt tactcatata tactttagat tgatttaaaa cttcattttt 6240

aatttaaaag gatctaggtg aagatccttt ttgataatct catgaccaaa atcccttaac 6300

gtgagttttc gttccactga gcgtcagacc ccgtagaaaa gatcaaagga tcttcttgag 6360

atcctttttt tctgcgcgta atctgctgct tgcaaacaaa aaaaccaccg ctaccagcgg 6420

tggtttgttt gccggatcaa gagctaccaa ctctttttcc gaaggtaact ggcttcagca 6480

gagcgcagat accaaatact gttcttctag tgtagccgta gttaggccac cacttcaaga 6540

actctgtagc accgcctaca tacctcgctc tgctaatcct gttaccagtg gctgctgcca 6600

gtggcgataa gtcgtgtctt accgggttgg actcaagacg atagttaccg gataaggcgc 6660

agcggtcggg ctgaacgggg ggttcgtgca cacagcccag cttggagcga acgacctaca 6720

ccgaactgag atacctacag cgtgagctat gagaaagcgc cacgcttccc gaagggagaa 6780

aggcggacag gtatccggta agcggcaggg tcggaacagg agagcgcacg agggagcttc 6840

cagggggaaa cgcctggtat ctttatagtc ctgtcgggtt tcgccacctc tgacttgagc 6900

gtcgattttt gtgatgctcg tcaggggggc ggagcctatg gaaaaacgcc agcaacgcgg 6960

cctttttacg gttcctggcc ttttgctggc cttttgctca catgttcttt cctgcgttat 7020

cccctgattc tgtggataac cgtattaccg cctttgagtg agctgatacc gctcgccgca 7080

gccgaacgac cgagcgcagc gagtcagtga gcgaggaagc ggaagagcgc ccaatacgca 7140

aaccgcctct ccccgcgcgt tggccgattc attaatgcag ctggcacgac aggtttcccg 7200

actggaaagc gggcagtgag cgcaacgcaa ttaatgtgag ttagctcact cattaggcac 7260

cccaggcttt acactttatg cttccggctc gtatgttgtg tggaattgtg agcggataac 7320

aatttcacac aggaaacagc tatgaccatg attacgccaa 7360

<210> 7
<211> 7330
<212> DNA
<213> Artificial Sequence

<400> 7
gcgcgccgat atcgttaacg ccccgcgccg gccgctctag aactagtgga tcccccggaa 60

gatcagaagt tcctattccg aagttcctat tctctagaaa gtataggaac ttctgatctg 120

cgcagccgcc atgccggggt tttacgagat tgtgattaag gtccccagcg accttgacga 180

gcatctgccc ggcatttctg acagctttgt gaactgggtg gccgagaagg aatgggagtt 240

gccgccagat tctgacatgg atctgaatct gattgagcag gcacccctga ccgtggccga 300

gaagctgcag cgcgactttc tgacggaatg gcgccgtgtg agtaaggccc cggaggccct 360

tttctttgtg caatttgaga agggagagag ctacttccac atgcacgtgc tcgtggaaac 420

caccggggtg aaatccatgg ttttgggacg tttcctgagt cagattcgcg aaaaactgat 480

tcagagaatt taccgcggga tcgagccgac tttgccaaac tggttcgcgg tcacaaagac 540

cagaaatggc gccggaggcg ggaacaaggt ggtggatgag tgctacatcc ccaattactt 600

gctccccaaa acccagcctg agctccagtg ggcgtggact aatatggaac agtatttaag 660

cgcctgtttg aatctcacgg agcgtaaacg gttggtggcg cagcatctga cgcacgtgtc 720

gcagacgcag gagcagaaca aagagaatca gaatcccaat tctgatgcgc cggtgatcag 780

atcaaaaact tcagccaggt acatggagct ggtcgggtgg ctcgtggaca aggggattac 840

ctcggagaag cagtggatcc aggaggacca ggcctcatac atctccttca atgcggcctc 900

caactcgcgg tcccaaatca aggctgcctt ggacaatgcg ggaaagatta tgagcctgac 960

taaaaccgcc cccgactacc tggtgggcca gcagcccgtg gaggacattt ccagcaatcg 1020

gatttataaa attttggaac taaacgggta cgatccccaa tatgcggctt ccgtctttct 1080

gggatgggcc acgaaaaagt tcggcaagag gaacaccatc tggctgtttg ggcctgcaac 1140

taccgggaag accaacatcg cggaggccat agcccacact gtgcccttct acgggtgcgt 1200

aaactggacc aatgagaact ttcccttcaa cgactgtgtc gacaagatgg tgatctggtg 1260

ggaggagggg aagatgaccg ccaaggtcgt ggagtcggcc aaagccattc tcggaggaag 1320

caaggtgcgc gtggaccaga aatgcaagtc ctcggcccag atagacccga ctcccgtgat 1380

cgtcacctcc aacaccaaca tgtgcgccgt gattgacggg aactcaacga ccttcgaaca 1440

ccagcagccg ttgcaagacc ggatgttcaa atttgaactc acccgccgtc tggatcatga 1500

ctttgggaag gtcaccaagc aggaagtcaa agactttttc cggtggcaaa aggatcacgt 1560

ggttgaggtg gagcatgaat tctacgtcaa aaagggtgga gccaagaaaa gacccgcccc 1620

cagtgacgca gatataagtg agcccaaacg ggtgcgcgag tcagttgcgc agccatcgac 1680

gtcagacgcg gaagcttcga tcaactacgc agacaggtac caaaacaaat gttctcgtca 1740

cgtgggcatg aatctgatgc tgtttccctg cagacaatgc gagagaatga atcagaattc 1800

aaatatctgc ttcactcacg gacagaaaga ctgtttagag tgctttcccg tgtcagaatc 1860

tcaacccgtt tctgtcgtca aaaaggcgta tcagaaactg tgctacattc atcatatcat 1920

gggaaaggtg ccagacgctt gcactgcctg cgatctggtc aatgtggatt tggatgactg 1980

catctttgaa caataaatga tttaaatcag gtatggctgc cgatggttat cttccagatt 2040

ggctcgagga caacctctct gagggcattc gcgagtggtg ggacttgaaa cctggagccc 2100

cgaagcccaa agccaaccag caaaagcagg acgacggccg gggtctggtg cttcctggct 2160

acaagtacct cggacccttc aacggactcg acaaggggga gcccgtcaac gcggcggacg 2220

cagcggccct cgagcacgac aaggcctacg accagcagct caaagcgggt gacaatccgt 2280

acctgcggta taaccacgcc gacgccgagt ttcaggagcg tctgcaagaa gatacgtctt 2340

ttgggggcaa cctcgggcga gcagtcttcc aggccaagaa gcgggttctc gaacctctcg 2400

gtctggttga ggaaggcgct aagacggctc ctggaaagaa acgtccggta gagcagtcgc 2460

cacaagagcc agactcctcc tcgggcatcg gcaagacagg ccagcagccc gctaaaaaga 2520

gactcaattt tggtcagact ggcgactcag agtcagtccc cgatccacaa cctctcggag 2580

aacctccagc aacccccgct gctgtgggac ctactacaat ggcttcaggc ggtggcgcac 2640

caatggcaga caataacgaa ggcgccgacg gagtgggtaa tgcctcagga aattggcatt 2700

gcgattccac atggctgggc gacagagtca tcaccaccag cacccgcacc tgggccttgc 2760

ccacctacaa taaccacctc tacaagcaaa tctccagtgc ttcaacgggg gccagcaacg 2820

acaaccacta cttcggctac agcaccccct gggggtattt tgatttcaac agattccact 2880

gccacttttc accacgtgac tggcagcgac tcatcaacaa caattgggga ttccggccca 2940

agagactcaa cttcaaactc ttcaacatcc aagtcaagga ggtcacgacg aatgatggcg 3000

tcacaaccat cgctaataac cttaccagca cggttcaagt cttctcggac tcggagtacc 3060

agcttccgta cgtcctcggc tctgcgcacc agggctgcct ccctccgttc ccggcggacg 3120

tgttcatgat tccgcaatac ggctacctga cgctcaacaa tggcagccaa gccgtgggac 3180

gttcatcctt ttactgcctg gaatatttcc cttctcagat gctgagaacg ggcaacaact 3240

ttaccttcag ctacaccttt gaggaagtgc ctttccacag cagctacgcg cacagccaga 3300

gcctggaccg gctgatgaat cctctcatcg accaatacct gtattacctg aacagaactc 3360

aaaatcagtc cggaagtgcc caaaacaagg acttgctgtt tagccgtggg tctccagctg 3420

gcatgtctgt tcagcccaaa aactggctac ctggaccctg ttatcggcag cagcgcgttt 3480

ctaaaacaaa aacagacaac aacaacagca attttacctg gactggtgct tcaaaatata 3540

acctcaatgg gcgtgaatcc atcatcaacc ctggcactgc tatggcctca cacaaagacg 3600

acgaagacaa gttctttccc atgagcggtg tcatgatttt tggaaaagag agcgccggag 3660

cttcaaacac tgcattggac aatgtcatga ttacagacga agaggaaatt aaagccacta 3720

accctgtggc caccgaaaga tttgggaccg tggcagtcaa tttccagagc agcagcacag 3780

accctgcgac cggagatgtg catgctatgg gagcattacc tggcatggtg tggcaagata 3840

gagacgtgta cctgcagggt cccatttggg ccaaaattcc tcacacagat ggacactttc 3900

acccgtctcc tcttatgggc ggctttggac tcaagaaccc gcctcctcag atcctcatca 3960

aaaacacgcc tgttcctgcg aatcctccgg cggagttttc agctacaaag tttgcttcat 4020

tcatcaccca atactccaca ggacaagtga gtgtggaaat tgaatgggag ctgcagaaag 4080

aaaacagcaa gcgctggaat cccgaagtgc agtacacatc caattatgca aaatctgcca 4140

acgttgattt tactgtggac aacaatggac tttatactga gcctcgcccc attggcaccc 4200

gttaccttac ccgtcccctg taattgcttg ttaatcaata aaccgtttaa ttcgtttcag 4260

ttgaactttg gtctctgcgt atttctttct tatctagttt ccatggctac gtagataagt 4320

agcatggcgg gttaatcatt aactacagcc cgggcgttta aacagcgggc ggaggggtgg 4380

agtcgtgacg tgaattacgt catagggtta gggaggtcct gtattagagg tcacgtgagt 4440

gttttgcgac attttgcgac accatgtggt ctcgctgggg ggggggcccc gagtgagcac 4500

gcagggtctc cattttgaag cgggaggttt gaacgagcgc tggcgcgctc actggccgtc 4560

gttttacaac gtcgtgactg ggaaaaccct ggcgttaccc aacttaatcg ccttgcagca 4620

catccccctt tcgccagctg gcgtaatagc gaagaggccc gcaccgatcg cccttcccaa 4680

cagttgcgca gcctgaatgg cgaatggaaa ttgtaagcgt taatattttg ttaaaattcg 4740

cgttaaattt ttgttaaatc agctcatttt tttaaccaat aggccgaaat cggcaaaatc 4800

ccttataaat caaaagaata gaccgagata gggttgagtg ttgttccagt ttggaacaag 4860

agtccactat taagaacgtg gactccaacg tcaaagggcg aaaaaccgtc tatcagggcg 4920

atggcccact acgtgaacca tcaccctaat caagtttttt ggggtcgagg tgccgtaaag 4980

cactaaatcg gaaccctaaa gggagccccc gatttagagc ttgacgggga aagccggcga 5040

acgtggcgag aaaggaaggg aagaaagcga aaggagcggg cgctagggcg ctggcaagtg 5100

tagcggtcac gctgcgcgta accaccacac ccgccgcgct taatgcgccg ctacagggcg 5160

cgtcaggtgg cacttttcgg ggaaatgtgc gcggaacccc tatttgttta tttttctaaa 5220

tacattcaaa tatgtatccg ctcatgagac aataaccctg ataaatgctt caataatatt 5280

gaaaaaggaa gagtatgagt attcaacatt tccgtgtcgc ccttattccc ttttttgcgg 5340

cattttgcct tcctgttttt gctcacccag aaacgctggt gaaagtaaaa gatgctgaag 5400

atcagttggg tgcacgagtg ggttacatcg aactggatct caacagcggt aagatccttg 5460

agagttttcg ccccgaagaa cgttttccaa tgatgagcac ttttaaagtt ctgctatgtg 5520

gcgcggtatt atcccgtatt gacgccgggc aagagcaact cggtcgccgc atacactatt 5580

ctcagaatga cttggttgag tactcaccag tcacagaaaa gcatcttacg gatggcatga 5640

cagtaagaga attatgcagt gctgccataa ccatgagtga taacactgcg gccaacttac 5700

ttctgacaac gatcggagga ccgaaggagc taaccgcttt tttgcacaac atgggggatc 5760

atgtaactcg ccttgatcgt tgggaaccgg agctgaatga agccatacca aacgacgagc 5820

gtgacaccac gatgcctgta gcaatggcaa caacgttgcg caaactatta actggcgaac 5880

tacttactct agcttcccgg caacaattaa tagactggat ggaggcggat aaagttgcag 5940

gaccacttct gcgctcggcc cttccggctg gctggtttat tgctgataaa tctggagccg 6000

gtgagcgtgg gtctcgcggt atcattgcag cactggggcc agatggtaag ccctcccgta 6060

tcgtagttat ctacacgacg gggagtcagg caactatgga tgaacgaaat agacagatcg 6120

ctgagatagg tgcctcactg attaagcatt ggtaactgtc agaccaagtt tactcatata 6180

tactttagat tgatttaaaa cttcattttt aatttaaaag gatctaggtg aagatccttt 6240

ttgataatct catgaccaaa atcccttaac gtgagttttc gttccactga gcgtcagacc 6300

ccgtagaaaa gatcaaagga tcttcttgag atcctttttt tctgcgcgta atctgctgct 6360

tgcaaacaaa aaaaccaccg ctaccagcgg tggtttgttt gccggatcaa gagctaccaa 6420

ctctttttcc gaaggtaact ggcttcagca gagcgcagat accaaatact gttcttctag 6480

tgtagccgta gttaggccac cacttcaaga actctgtagc accgcctaca tacctcgctc 6540

tgctaatcct gttaccagtg gctgctgcca gtggcgataa gtcgtgtctt accgggttgg 6600

actcaagacg atagttaccg gataaggcgc agcggtcggg ctgaacgggg ggttcgtgca 6660

cacagcccag cttggagcga acgacctaca ccgaactgag atacctacag cgtgagctat 6720

gagaaagcgc cacgcttccc gaagggagaa aggcggacag gtatccggta agcggcaggg 6780

tcggaacagg agagcgcacg agggagcttc cagggggaaa cgcctggtat ctttatagtc 6840

ctgtcgggtt tcgccacctc tgacttgagc gtcgattttt gtgatgctcg tcaggggggc 6900

ggagcctatg gaaaaacgcc agcaacgcgg cctttttacg gttcctggcc ttttgctggc 6960

cttttgctca catgttcttt cctgcgttat cccctgattc tgtggataac cgtattaccg 7020

cctttgagtg agctgatacc gctcgccgca gccgaacgac cgagcgcagc gagtcagtga 7080

gcgaggaagc ggaagagcgc ccaatacgca aaccgcctct ccccgcgcgt tggccgattc 7140

attaatgcag ctggcacgac aggtttcccg actggaaagc gggcagtgag cgcaacgcaa 7200

ttaatgtgag ttagctcact cattaggcac cccaggcttt acactttatg cttccggctc 7260

gtatgttgtg tggaattgtg agcggataac aatttcacac aggaaacagc tatgaccatg 7320

attacgccaa 7330

<210> 8
<211> 7330
<212> DNA
<213> Artificial Sequence

<400> 8
gcgcgccgat atcgttaacg ccccgcgccg gccgctctag aactagtgga tcccccggaa 60

gatcagaagt tcctattccg aagttcctat tctctagaaa gtataggaac ttctgatctg 120

cgcagccgcc atgccggggt tttacgagat tgtgattaag gtccccagcg accttgacga 180

gcatctgccc ggcatttctg acagctttgt gaactgggtg gccgagaagg aatgggagtt 240

gccgccagat tctgacatgg atctgaatct gattgagcag gcacccctga ccgtggccga 300

gaagctgcag cgcgactttc tgacggaatg gcgccgtgtg agtaaggccc cggaggccct 360

tttctttgtg caatttgaga agggagagag ctacttccac atgcacgtgc tcgtggaaac 420

caccggggtg aaatccatgg ttttgggacg tttcctgagt cagattcgcg aaaaactgat 480

tcagagaatt taccgcggga tcgagccgac tttgccaaac tggttcgcgg tcacaaagac 540

cagaaatggc gccggaggcg ggaacaaggt ggtggatgag tgctacatcc ccaattactt 600

gctccccaaa acccagcctg agctccagtg ggcgtggact aatatggaac agtatttaag 660

cgcctgtttg aatctcacgg agcgtaaacg gttggtggcg cagcatctga cgcacgtgtc 720

gcagacgcag gagcagaaca aagagaatca gaatcccaat tctgatgcgc cggtgatcag 780

atcaaaaact tcagccaggt acatggagct ggtcgggtgg ctcgtggaca aggggattac 840

ctcggagaag cagtggatcc aggaggacca ggcctcatac atctccttca atgcggcctc 900

caactcgcgg tcccaaatca aggctgcctt ggacaatgcg ggaaagatta tgagcctgac 960

taaaaccgcc cccgactacc tggtgggcca gcagcccgtg gaggacattt ccagcaatcg 1020

gatttataaa attttggaac taaacgggta cgatccccaa tatgcggctt ccgtctttct 1080

gggatgggcc acgaaaaagt tcggcaagag gaacaccatc tggctgtttg ggcctgcaac 1140

taccgggaag accaacatcg cggaggccat agcccacact gtgcccttct acgggtgcgt 1200

aaactggacc aatgagaact ttcccttcaa cgactgtgtc gacaagatgg tgatctggtg 1260

ggaggagggg aagatgaccg ccaaggtcgt ggagtcggcc aaagccattc tcggaggaag 1320

caaggtgcgc gtggaccaga aatgcaagtc ctcggcccag atagacccga ctcccgtgat 1380

cgtcacctcc aacaccaaca tgtgcgccgt gattgacggg aactcaacga ccttcgaaca 1440

ccagcagccg ttgcaagacc ggatgttcaa atttgaactc acccgccgtc tggatcatga 1500

ctttgggaag gtcaccaagc aggaagtcaa agactttttc cggtgggcaa aggatcacgt 1560

ggttgaggtg gagcatgaat tctacgtcaa aaagggtgga gccaagaaaa gacccgcccc 1620
cagtgacgca gatataagtg agcccaaacg ggtgcgcgag tcagttgcgc agccatcgac 1680
gtcagacgcg gaagcttcga tcaactacgc agacaggtac caaaacaaat gttctcgtca 1740
cgtgggcatg aatctgatgc tgtttccctg cagacaatgc gagagaatga atcagaattc 1800
aaatatctgc ttcactcacg gacagaaaga ctgtttagag tgctttcccg tgtcagaatc 1860
tcaacccgtt tctgtcgtca aaaaggcgta tcagaaactg tgctacattc atcatatcat 1920
gggaaaggtg ccagacgctt gcactgcctg cgatctggtc aatgtggatt tggatgactg 1980
catctttgaa caataaatga tttaaatcag gtatggctgc cgatggttat cttccagatt 2040
ggctcgagga caacctctct gagggcattc gcgagtggtg ggacttgaaa cctggagccc 2100
cgaaacccaa agccaaccag caaaagcagg acgacggccg gggtctggtg cttcctggct 2160
acaagtacct cggacccttc aacggactcg acaaggggga gcccgtcaac gcggcggatg 2220
cagcggccct cgagcacgac aaggcctacg accagcagct caaagcgggt gacaatccgt 2280
acctgcggta taaccacgcc gacgccgagt ttcaggagcg tctgcaagaa gatacgtctt 2340
ttgggggcaa cctcgggcga gcagtcttcc aggccaagaa gagggttctc gaaccttttg 2400
gtctggttga ggaaggtgct aagacggctc ctggaaagaa acgtccggta gagcagtcgc 2460
cacaagagcc agactcctcc tcgggcattg gcaagacagg ccagcagccc gctaaaaaga 2520
gactcaattt tggtcagact ggcgactcag agtcagtccc cgacccacaa cctctcggag 2580
aacctccagc aacccccgct gctgtgggac ctactacaat ggcttcaggc ggtggcgcac 2640
caatggcaga caataacgaa ggcgccgacg gagtgggtaa tgcctcagga aattggcatt 2700
gcgattccac atggctgggc gacagagtca tcaccaccag cacccgaaca tgggccttgc 2760
ccacctataa caaccacctc tacaagcaaa tctccagtgc ttcaacgggg gccagcaacg 2820
acaaccacta cttcggctac agcaccccct gggggtattt tgatttcaac agattccact 2880
gccatttctc accacgtgac tggcagcgac tcatcaacaa caattgggga ttccggccca 2940
agagactcaa cttcaagctc ttcaacatcc aagtcaagga ggtcacgacg aatgatggcg 3000
tcacgaccat cgctaataac cttaccagca cggttcaagt cttctcggac tcggagtacc 3060
agttgccgta cgtcctcggc tctgcgcacc agggctgcct ccctccgttc ccggcggacg 3120
tgttcatgat tccgcagtac ggctacctaa cgctcaacaa tggcagccag gcagtgggac 3180
ggtcatcctt ttactgcctg gaatatttcc catcgcagat gctgagaacg ggcaataact 3240
ttaccttcag ctacaccttc gaggacgtgc ctttccacag cagctacgcg cacagccaga 3300
gcctggaccg gctgatgaat cctctcatcg accagtacct gtattacctg aacagaactc 3360
agaatcagtc cggaagtgcc caaaacaagg acttgctgtt tagccggggg tctccagctg 3420
gcatgtctgt tcagcccaaa aactggctac ctggaccctg ttaccggcag cagcgcgttt 3480

ctaaaacaaa aacagacaac aacaacagca actttacctg gactggtgct tcaaaatata 3540

accttaatgg gcgtgaatct ataatcaacc ctggcactgc tatggcctca cacaaagacg 3600

acaaagacaa gttctttccc atgagcggtg tcatgatttt tggaaaggag agcgccggag 3660

cttcaaacac tgcattggac aatgtcatga tcacagacga agaggaaatc aaagccacta 3720

accccgtggc caccgaaaga tttgggactg tggcagtcaa tctccagagc agcagcacag 3780

accctgcgac cggagatgtg catgttatgg gagccttacc tggaatggtg tggcaagaca 3840

gagacgtata cctgcagggt cctatttggg ccaaaattcc tcacacggat ggacactttc 3900

acccgtctcc tctcatgggc ggctttggac ttaagcaccc gcctcctcag atcctcatca 3960

aaaacacgcc tgttcctgcg aatcctccgg cagagttttc ggctacaaag tttgcttcat 4020

tcatcaccca gtattccaca ggacaagtga gcgtggagat tgaatgggag ctgcagaaag 4080

aaaacagcaa acgctggaat cccgaagtgc agtatacatc taactatgca aaatctgcca 4140

acgttgattt cactgtggac aacaatggac tttatactga gcctcgcccc attggcaccc 4200

gttacctcac ccgtcccctg taattgcttg ttaatcaata aaccgtttaa ttcgtttcag 4260

ttgaactttg gtctctgcgt atttctttct tatctagttt ccatggctac gtagataagt 4320

agcatggcgg gttaatcatt aactacagcc cgggcgttta aacagcgggc ggaggggtgg 4380

agtcgtgacg tgaattacgt catagggtta gggaggtcct gtattagagg tcacgtgagt 4440

gttttgcgac attttgcgac accatgtggt ctcgctgggg ggggggcccc gagtgagcac 4500

gcagggtctc cattttgaag cgggaggttt gaacgagcgc tggcgcgctc actggccgtc 4560

gttttacaac gtcgtgactg ggaaaaccct ggcgttaccc aacttaatcg ccttgcagca 4620

catccccctt tcgccagctg gcgtaatagc gaagaggccc gcaccgatcg cccttcccaa 4680

cagttgcgca gcctgaatgg cgaatggaaa ttgtaagcgt taatattttg ttaaaattcg 4740

cgttaaattt ttgttaaatc agctcatttt ttaaccaata ggccgaaatc ggcaaaatcc 4800

cttataaatc aaaagaatag accgagatag ggttgagtgt tgttccagtt tggaacaaga 4860

gtccactatt aaagaacgtg gactccaacg tcaaagggcg aaaaaccgtc tatcagggcg 4920

atggcccact acgtgaacca tcaccctaat caagtttttt ggggtcgagg tgccgtaaag 4980

cactaaatcg gaaccctaaa gggagccccc gatttagagc ttgacgggga aagccggcga 5040

acgtggcgag aaaggaaggg aagaaagcga aaggagcggg cgctagggcg ctggcaagtg 5100

tagcggtcac gctgcgcgta accaccacac ccgccgcgct taatgcgccg ctacagggcg 5160

cgtcaggtgg cacttttcgg ggaaatgtgc gcggaacccc tatttgttta tttttctaaa 5220

tacattcaaa tatgtatccg ctcatgagac aataaccctg ataaatgctt caataatatt 5280

gaaaaaggaa gagtatgagt attcaacatt tccgtgtcgc ccttattccc ttttttgcgg 5340

cattttgcct tcctgttttt gctcacccag aaacgctggt gaaagtaaaa gatgctgaag 5400

atcagttggg tgcacgagtg ggttacatcg aactggatct caacagcggt aagatccttg 5460

agagttttcg ccccgaagaa cgttttccaa tgatgagcac ttttaaagtt ctgctatgtg 5520

gcgcggtatt atcccgtatt gacgccgggc aagagcaact cggtcgccgc atacactatt 5580

ctcagaatga cttggttgag tactcaccag tcacagaaaa gcatcttacg gatggcatga 5640

cagtaagaga attatgcagt gctgccataa ccatgagtga taacactgcg gccaacttac 5700

ttctgacaac gatcggagga ccgaaggagc taaccgcttt tttgcacaac atgggggatc 5760

atgtaactcg ccttgatcgt tgggaaccgg agctgaatga agccatacca aacgacgagc 5820

gtgacaccac gatgcctgta gcaatggcaa caacgttgcg caaactatta actggcgaac 5880

tacttactct agcttcccgg caacaattaa tagactggat ggaggcggat aaagttgcag 5940

gaccacttct gcgctcggcc cttccggctg gctggtttat tgctgataaa tctggagccg 6000

gtgagcgtgg gtctcgcggt atcattgcag cactggggcc agatggtaag ccctcccgta 6060

tcgtagttat ctacacgacg gggagtcagg caactatgga tgaacgaaat agacagatcg 6120

ctgagatagg tgcctcactg attaagcatt ggtaactgtc agaccaagtt tactcatata 6180

tactttagat tgatttaaaa cttcattttt aatttaaaag gatctaggtg aagatccttt 6240

ttgataatct catgaccaaa atcccttaac gtgagttttc gttccactga gcgtcagacc 6300

ccgtagaaaa gatcaaagga tcttcttgag atcctttttt tctgcgcgta atctgctgct 6360

tgcaaacaaa aaaaccaccg ctaccagcgg tggtttgttt gccggatcaa gagctaccaa 6420

ctctttttcc gaaggtaact ggcttcagca gagcgcagat accaaatact gttcttctag 6480

tgtagccgta gttaggccac cacttcaaga actctgtagc accgcctaca tacctcgctc 6540

tgctaatcct gttaccagtg gctgctgcca gtggcgataa gtcgtgtctt accgggttgg 6600

actcaagacg atagttaccg gataaggcgc agcggtcggg ctgaacgggg ggttcgtgca 6660

cacagcccag cttggagcga acgacctaca ccgaactgag atacctacag cgtgagctat 6720

gagaaagcgc cacgcttccc gaagggagaa aggcggacag gtatccggta agcggcaggg 6780

tcggaacagg agagcgcacg agggagcttc cagggggaaa cgcctggtat ctttatagtc 6840

ctgtcgggtt tcgccacctc tgacttgagc gtcgattttt gtgatgctcg tcaggggggc 6900

ggagcctatg gaaaaacgcc agcaacgcgg cctttttacg gttcctggcc ttttgctggc 6960

cttttgctca catgttcttt cctgcgttat cccctgattc tgtggataac cgtattaccg 7020

cctttgagtg agctgatacc gctcgccgca gccgaacgac cgagcgcagc gagtcagtga 7080

gcgaggaagc ggaagagcgc ccaatacgca aaccgcctct ccccgcgcgt tggccgattc 7140

attaatgcag ctggcacgac aggtttcccg actggaaagc gggcagtgag cgcaacgcaa 7200

ttaatgtgag ttagctcact cattaggcac cccaggcttt acactttatg cttccggctc 7260

gtatgttgtg tggaattgtg agcggataac aatttcacac aggaaacagc tatgaccatg 7320

attacgccaa 7330

<210> 9
<211> 7330
<212> DNA
<213> Artificial Sequence

<400> 9
gcgcgccgat atcgttaacg ccccgcgccg gccgctctag aactagtgga tcccccggaa 60

gatcagaagt tcctattccg aagttcctat tctctagaaa gtataggaac ttctgatctg 120

cgcagccgcc atgccggggt tttacgagat tgtgattaag gtccccagcg accttgacga 180

gcatctgccc ggcatttctg acagctttgt gaactgggtg gccgagaagg aatgggagtt 240

gccgccagat tctgacatgg atctgaatct gattgagcag gcacccctga ccgtggccga 300

gaagctgcag cgcgactttc tgacggaatg gcgccgtgtg agtaaggccc cggaggccct 360

tttctttgtg caatttgaga agggagagag ctacttccac atgcacgtgc tcgtggaaac 420

caccggggtg aaatccatgg ttttgggacg tttcctgagt cagattcgcg aaaaactgat 480

tcagagaatt taccgcggga tcgagccgac tttgccaaac tggttcgcgg tcacaaagac 540

cagaaatggc gccggaggcg ggaacaaggt ggtggatgag tgctacatcc ccaattactt 600

gctccccaaa acccagcctg agctccagtg ggcgtggact aatatggaac agtatttaag 660

cgcctgtttg aatctcacgg agcgtaaacg gttggtggcg cagcatctga cgcacgtgtc 720

gcagacgcag gagcagaaca aagagaatca gaatcccaat tctgatgcgc cggtgatcag 780

atcaaaaact tcagccaggt acatggagct ggtcgggtgg ctcgtggaca aggggattac 840

ctcggagaag cagtggatcc aggaggacca ggcctcatac atctccttca atgcggcctc 900

caactcgcgg tcccaaatca aggctgcctt ggacaatgcg ggaaagatta tgagcctgac 960

taaaaccgcc cccgactacc tggtgggcca gcagcccgtg gaggacattt ccagcaatcg 1020

gatttataaa attttggaac taaacgggta cgatccccaa tatgcggctt ccgtctttct 1080

gggatgggcc acgaaaaagt tcggcaagag gaacaccatc tggctgtttg ggcctgcaac 1140

taccgggaag accaacatcg cggaggccat agcccacact gtgcccttct acgggtgcgt 1200

aaactggacc aatgagaact ttcccttcaa cgactgtgtc gacaagatgg tgatctggtg 1260

ggaggagggg aagatgaccg ccaaggtcgt ggagtcggcc aaagccattc tcggaggaag 1320

caaggtgcgc gtggaccaga aatgcaagtc ctcggcccag atagacccga ctcccgtgat 1380

cgtcacctcc aacaccaaca tgtgcgccgt gattgacggg aactcaacga ccttcgaaca 1440

ccagcagccg ttgcaagacc ggatgttcaa atttgaactc acccgccgtc tggatcatga 1500

ctttgggaag gtcaccaagc aggaagtcaa agactttttc cggtgggcaa aggatcacgt 1560

ggttgaggtg gagcatgaat tctacgtcaa aaaggtggga gccaagaaaa gacccgcccc 1620

cagtgacgca gatataagtg agcccaaacg ggtgcgcgag tcagttgcgc agccatcgac 1680

gtcagacgcg gaagcttcga tcaactacgc agacaggtac caaaacaaat gttctcgtca 1740

cgtgggcatg aatctgatgc tgtttccctg cagacaatgc gagagaatga atcagaattc 1800

aaatatctgc ttcactcacg gacagaaaga ctgtttagag tgctttcccg tgtcagaatc 1860

tcaacccgtt tctgtcgtca aaaaggcgta tcagaaactg tgctacattc atcatatcat 1920

gggaaaggtg ccagacgctt gcactgcctg cgatctggtc aatgtggatt tggatgactg 1980

catctttgaa caataaatga tttaaatcag gtatggctgc cgatggttat cttccagatt 2040

ggctcgagga caaccttagt gaaggaattc gcgagtggtg ggctttgaaa cctggagccc 2100

ctcaacccaa ggcaaatcaa caacatcaag acaacgctcg aggtcttgtg cttccgggtt 2160

acaaatacct tggacccggc aacggactcg acaaggggga gccggtcaac gcagcagacg 2220

cggcggccct cgagcacgac aaggcctacg accagcagct caaggccgga gacaacccgt 2280

acctcaagta caaccacgcc gacgccgagt tccaggagcg gctcaaagaa gatacgtctt 2340

ttgggggcaa cctcgggcga gcagtcttcc aggccaaaaa gaggcttctt gaacctcttg 2400

gtctggttga ggaagcggct aagacggctc ctggaaagaa gaggcctgta gagcagtctc 2460

ctcaggaacc ggactcctcc gcgggtattg gcaaatcggg tgcacagccc gctaaaaaga 2520

gactcaattt cggtcagact ggcgacacag agtcagtccc agaccctcaa ccaatcggag 2580

aacctcccgc agccccctca ggtgtgggat ctcttacaat ggcttcaggt ggtggcgcac 2640

cagtggcaga caataacgaa ggtgccgatg gagtgggtag ttcctcggga aattggcatt 2700

gcgattccca atggctgggg gacagagtca tcaccaccag cacccgaacc tgggccctgc 2760

ccacctacaa caatcacctc tacaagcaaa tctccaacag cacatctgga ggatcttcaa 2820

atgacaacgc ctacttcggc tacagcaccc cctgggggta ttttgacttc aacagattcc 2880

actgccactt ctcaccacgt gactggcagc gactcatcaa caacaactgg ggattccggc 2940

ctaagcgact caacttcaag ctcttcaaca ttcaggtcaa agaggttacg gacaacaatg 3000

gagtcaagac catcgccaat aaccttacca gcacggtcca ggtcttcacg gactcagact 3060

atcagctccc gtacgtgctc gggtcggctc acgagggctg cctcccgccg ttcccagcgg 3120

acgttttcat gattcctcag tacgggtatc tgacgcttaa tgatggaagc caggccgtgg 3180

gtcgttcgtc cttttactgc ctggaatatt tcccgtcgca aatgctaaga acgggtaaca 3240

acttccagtt cagctacgag tttgagaacg tacctttcca tagcagctac gctcacagcc 3300

aaagcctgga ccgactaatg aatccactca tcgaccaata cttgtactat ctctcaaaga 3360

ctattaacgg ttctggacag aatcaacaaa cgctaaaatt cagtgtggcc ggacccagca 3420

acatggctgt ccagggaaga aactacatac ctggacccag ctaccgacaa caacgtgtct 3480

caaccactgt gactcaaaac aacaacagcg aatttgcttg gcctggagct tcttcttggg 3540

ctctcaatgg acgtaatagc ttgatgaatc ctggacctgc tatggccagc cacaaagaag 3600

gagaggaccg tttctttcct ttgtctggat ctttaatttt tggcaaacaa ggaactggaa 3660

gagacaacgt ggatgcggac aaagtcatga taaccaacga agaagaaatt aaaactacta 3720

acccggtagc aacggagtcc tatggacaag tggccacaaa ccaccagagt gcccaagcac 3780

aggcgcagac cggctgggtt caaaaccaag gaatacttcc gggtatggtt tggcaggaca 3840

gagatgtgta cctgcaagga cccatttggg ccaaaattcc tcacacggac ggcaactttc 3900

acccttctcc gctgatggga gggtttggaa tgaagcaccc gcctcctcag atcctcatca 3960

aaaacacacc tgtacctgcg gatcctccaa cggccttcaa caaggacaag ctgaactctt 4020

tcatcaccca gtattctact ggccaagtca gcgtggagat cgagtgggag ctgcagaagg 4080

aaaacagcaa gcgctggaac ccggagatcc agtacacttc caactattac aagtctaata 4140

atgttgaatt tgctgttaat actgaaggtg tatatagtga accccgcccc attggcacca 4200

gatacctgac tcgtaatctg taattgcttg ttaatcaata aaccgtttaa ttcgtttcag 4260

ttgaactttg gtctctgcgt atttctttct tatctagttt ccatggctac gtagataagt 4320

agcatggcgg gttaatcatt aactacagcc cgggcgttta aacagcgggc ggaggggtgg 4380

agtcgtgacg tgaattacgt catagggtta gggaggtcct gtattagagg tcacgtgagt 4440

gttttgcgac attttgcgac accatgtggt ctcgctgggg ggggggcccc gagtgagcac 4500

gcagggtctc cattttgaag cgggaggttt gaacgagcgc tggcgcgctc actggccgtc 4560

gttttacaac gtcgtgactg ggaaaaccct ggcgttaccc aacttaatcg ccttgcagca 4620

catccccctt tcgccagctg gcgtaatagc gaagaggccc gcaccgatcg cccttcccaa 4680

cagttgcgca gcctgaatgg cgaatggaaa ttgtaagcgt taatattttg ttaaaattcg 4740

cgttaaattt ttgttaaatc agctcatttt tttaaccaat aggccgaaat cggcaaaatc 4800

ccttataaat caaaagaata gaccgagata gggttgagtg ttgttccagt ttggaacaag 4860

agtccactat taagaacgtg gactccaacg tcaaagggcg aaaaaccgtc tatcagggcg 4920

atggcccact acgtgaacca tcaccctaat caagtttttt ggggtcgagg tgccgtaaag 4980

cactaaatcg gaaccctaaa gggagccccc gatttagagc ttgacgggga aagccggcga 5040

acgtggcgag aaaggaaggg aagaaagcga aaggagcggg cgctagggcg ctggcaagtg 5100

tagcggtcac gctgcgcgta accaccacac ccgccgcgct taatgcgccg ctacagggcg 5160

cgtcaggtgg cacttttcgg ggaaatgtgc gcggaacccc tatttgttta tttttctaaa 5220

tacattcaaa tatgtatccg ctcatgagac aataaccctg ataaatgctt caataatatt 5280

gaaaaaggaa gagtatgagt attcaacatt tccgtgtcgc ccttattccc ttttttgcgg 5340

cattttgcct tcctgttttt gctcacccag aaacgctggt gaaagtaaaa gatgctgaag 5400

atcagttggg tgcacgagtg ggttacatcg aactggatct caacagcggt aagatccttg 5460

agagttttcg ccccgaagaa cgttttccaa tgatgagcac ttttaaagtt ctgctatgtg 5520

gcgcggtatt atcccgtatt gacgccgggc aagagcaact cggtcgccgc atacactatt 5580

ctcagaatga cttggttgag tactcaccag tcacagaaaa gcatcttacg gatggcatga 5640

cagtaagaga attatgcagt gctgccataa ccatgagtga taacactgcg gccaacttac 5700

ttctgacaac gatcggagga ccgaaggagc taaccgcttt tttgcacaac atgggggatc 5760

atgtaactcg ccttgatcgt tgggaaccgg agctgaatga agccatacca aacgacgagc 5820

gtgacaccac gatgcctgta gcaatggcaa caacgttgcg caaactatta actggcgaac 5880

tacttactct agcttcccgg caacaattaa tagactggat ggaggcggat aaagttgcag 5940

gaccacttct gcgctcggcc cttccggctg gctggtttat tgctgataaa tctggagccg 6000

gtgagcgtgg gtctcgcggt atcattgcag cactggggcc agatggtaag ccctcccgta 6060

tcgtagttat ctacacgacg gggagtcagg caactatgga tgaacgaaat agacagatcg 6120

ctgagatagg tgcctcactg attaagcatt ggtaactgtc agaccaagtt tactcatata 6180

tactttagat tgatttaaaa cttcattttt aatttaaaag gatctaggtg aagatccttt 6240

ttgataatct catgaccaaa atcccttaac gtgagttttc gttccactga gcgtcagacc 6300

ccgtagaaaa gatcaaagga tcttcttgag atcctttttt tctgcgcgta atctgctgct 6360

tgcaaacaaa aaaaccaccg ctaccagcgg tggtttgttt gccggatcaa gagctaccaa 6420

ctctttttcc gaaggtaact ggcttcagca gagcgcagat accaaatact gttcttctag 6480

tgtagccgta gttaggccac cacttcaaga actctgtagc accgcctaca tacctcgctc 6540

tgctaatcct gttaccagtg gctgctgcca gtggcgataa gtcgtgtctt accgggttgg 6600

actcaagacg atagttaccg gataaggcgc agcggtcggg ctgaacgggg ggttcgtgca 6660

cacagcccag cttggagcga acgacctaca ccgaactgag atacctacag cgtgagctat 6720

gagaaagcgc cacgcttccc gaagggagaa aggcggacag gtatccggta agcggcaggg 6780

tcggaacagg agagcgcacg agggagcttc cagggggaaa cgcctggtat ctttatagtc 6840

ctgtcgggtt tcgccacctc tgacttgagc gtcgattttt gtgatgctcg tcaggggggc 6900

ggagcctatg gaaaaacgcc agcaacgcgg cctttttacg gttcctggcc ttttgctggc 6960

cttttgctca catgttcttt cctgcgttat cccctgattc tgtggataac cgtattaccg 7020

cctttgagtg agctgatacc gctcgccgca gccgaacgac cgagcgcagc gagtcagtga 7080

gcgaggaagc ggaagagcgc ccaatacgca aaccgcctct ccccgcgcgt tggccgattc 7140

attaatgcag ctggcacgac aggtttcccg actggaaagc gggcagtgag cgcaacgcaa 7200

ttaatgtgag ttagctcact cattaggcac cccaggcttt acactttatg cttccggctc 7260

gtatgttgtg tggaattgtg agcggataac aatttcacac aggaaacagc tatgaccatg 7320

attacgccaa 7330

<210> 10
<211> 7330
<212> DNA
<213> Artificial Sequence

<400> 10
gcgcgccgat atcgttaacg ccccgcgccg gccgctctag aactagtgga tcccccggaa 60

gatcagaagt tcctattccg aagttcctat tctctagaaa gtataggaac ttctgatctg 120

cgcagccgcc atgccggggt tttacgagat tgtgattaag gtccccagcg accttgacga 180

gcatctgccc ggcatttctg acagctttgt gaactgggtg gccgagaagg aatgggagtt 240

gccgccagat tctgacatgg atctgaatct gattgagcag gcacccctga ccgtggccga 300

gaagctgcag cgcgactttc tgacggaatg gcgccgtgtg agtaaggccc cggaggccct 360

tttctttgtg caatttgaga agggagagag ctacttccac atgcacgtgc tcgtggaaac 420

caccggggtg aaatccatgg ttttgggacg tttcctgagt cagattcgcg aaaaactgat 480

tcagagaatt taccgcggga tcgagccgac tttgccaaac tggttcgcgg tcacaaagac 540

cagaaatggc gccggaggcg ggaacaaggt ggtggatgag tgctacatcc ccaattactt 600

gctccccaaa acccagcctg agctccagtg ggcgtggact aatatggaac agtatttaag 660

cgcctgtttg aatctcacgg agcgtaaacg gttggtggcg cagcatctga cgcacgtgtc 720

gcagacgcag gagcagaaca aagagaatca gaatcccaat tctgatgcgc cggtgatcag 780

atcaaaaact tcagccaggt acatggagct ggtcgggtgg ctcgtggaca aggggattac 840

ctcggagaag cagtggatcc aggaggacca ggcctcatac atctccttca atgcggcctc 900

caactcgcgg tcccaaatca aggctgcctt ggacaatgcg ggaaagatta tgagcctgac 960

taaaaccgcc cccgactacc tggtgggcca gcagcccgtg gaggacattt ccagcaatcg 1020

gatttataaa attttggaac taaacgggta cgatccccaa tatgcggctt ccgtctttct 1080

gggatgggcc acgaaaaagt tcggcaagag gaacaccatc tggctgtttg ggcctgcaac 1140

taccgggaag accaacatcg cggaggccat agcccacact gtgcccttct acgggtgcgt 1200

aaactggacc aatgagaact ttcccttcaa cgactgtgtc gacaagatgg tgatctggtg 1260

ggaggagggg aagatgaccg ccaaggtcgt ggagtcggcc aaagccattc tcggaggaag 1320

caaggtgcgc gtggaccaga aatgcaagtc ctcggcccag atagacccga ctcccgtgat 1380

cgtcacctcc aacaccaaca tgtgcgccgt gattgacggg aactcaacga ccttcgaaca 1440

ccagcagccg ttgcaagacc ggatgttcaa atttgaactc acccgccgtc tggatcatga 1500

ctttgggaag gtcaccaagc aggaagtcaa agactttttc cggtgggcaa aggatcacgt 1560

ggttgaggtg gagcatgaat tctacgtcaa aaagggtgga gccaagaaaa gacccgcccc 1620

cagtgacgca gatataagtg agcccaaacg ggtgcgcgag tcagttgcgc agccatcgac 1680

gtcagacgcg gaagcttcga tcaactacgc agacaggtac caaaacaaat gttctcgtca 1740

cgtgggcatg aatctgatgc tgtttccctg cagacaatgc gagagaatga atcagaattc 1800

aaatatctgc ttcactcacg gacagaaaga ctgtttagag tgctttcccg tgtcagaatc 1860

tcaacccgtt tctgtcgtca aaaaggcgta tcagaaactg tgctacattc atcatatcat 1920

gggaaaggtg ccagacgctt gcactgcctg cgatctggtc aatgtggatt tggatgactg 1980

catctttgaa caataaatga tttaaatcag gtatggctgc cgatggttat cttccagatt 2040

ggctcgagga caacctctct gagggcattc gcgagtggtg ggacttgaaa cctggagccc 2100

cgaaacccaa agccaaccag caaaagcagg acgacggccg gggtctggtg cttcctggct 2160

acaagtacct cggacccttc aacggactcg acaaggggga gcccgtcaac gcggcggacg 2220

cagcggccct cgagcacgac aaggcctacg accagcagct caaagcgggt gacaatccgt 2280

acctgcggta taaccacgcc gacgccgagt ttcaggagcg tctgcaagaa gatacgtctt 2340

ttgggggcaa cctcgggcga gcagtcttcc aggccaagaa gcgggttctc gaacctctcg 2400

gtctggttga ggaaggcgct aagacggctc ctggaaagaa gagaccggta gagcaatcac 2460

cccaggaacc agactcctct tcgggcatcg gcaagaaagg ccagcagccc gcgagaaaga 2520

gactcaactt tgggcagact ggcgactcag agtcagtgcc cgaccctcaa ccactcggag 2580

aaccccccgc agccccctct ggtgtgggat ctaatacaat ggctgcaggc ggtggcgctc 2640

caatggcaga caataacgaa ggcgccgacg gagtgggtaa cgcctcagga aattggcatt 2700

gcgattccac atggctgggc gacagagtca tcaccaccag cacccgaacc tgggccctcc 2760

ccacctacaa caaccacctc tacaagcaaa tctccagcca atcgggaggc agcaccaacg 2820

acaacaccta cttcggctac agcaccccct gggggtattt tgactttaac agattccact 2880

gccacttctc accacgtgac tggcagcgac tcatcaacaa caactgggga ttccggccca 2940

agaagctcaa cttcaagctc ttcaacatcc aggtcaagga ggtcacgacg aatgatggca 3000

ccacgaccat cgccaataac cttaccagca cggttcaggt ctttacggac tcggaatacc 3060

agctcccgta cgtcctcggc tctgcgcacc agggctgcct gcctccgttc ccggcggacg 3120

tcttcatgat tcctcagtac gggtacctga ctctgaacaa tggcagtcag gccgtgggcc 3180

gttcctcctt ctactgcctg gaatactttc cttctcaaat gctgagaacg ggcaacaact 3240

ttcagttcag ctacacgttt gaggacgtgc cttttcacag cagctacgcg cacagccaaa 3300

gcctggaccg gctgatgaac cccctcatcg accagtacct gtactacctg tctcggactc 3360

agaccacgag tggtaccgca ggaaatcgga cgttgcaatt ttctcaggcc gggcctagta 3420

gcatggcgaa tcaggccaaa aactggctac ccgggccctg ctaccggcag caacgcgtct 3480

ccaagacaac caatcaaaat aacaacagca actttgcctg gaccggtgcc accaagtatc 3540

atctgaatgg cagagactct ctggtaaatc ccggtcccgc tatggcaacc cacaaggacg 3600

acgaagacaa attttttccg atgagcggag tcttaatatt tgggaaacag ggagctggaa 3660

atagcaacgt ggaccttgac aacgttatga taaccaacga ggaagaaatt aaaaccacca 3720

acccagtggc cacagaagag tacggcacgg tggccactaa cctgcaatcg gccaacaccg 3780

ctcctgctac agggaccgtc aacagtcaag gagccttacc tggcatggtc tggcaggacc 3840

gggacgtgta cctgcagggt cctatctggg ccaagattcc tcacacggac ggacactttc 3900

atccctcgcc gctgatggga ggctttggac tgaaacaccc gcctcctcag atcctgatta 3960

agaatacacc tgttcccgcg aatcctccaa ctaccttcag tccagctaag tttgcgtcgt 4020

tcatcacgca gtacagcacc ggacaggtca gcgtggaaat tgaatgggag ctgcagaaag 4080

aaaacagcaa acgctggaac ccagagattc aatacacttc caactacaac aaatctacaa 4140

atgtggactt tgctgttgac acaaatggcg tttattctga gcctcgcccc atcggcaccc 4200

gttacctcac ccgtaatctg taattgcttg ttaatcaata aaccgtttaa ttcgtttcag 4260

ttgaactttg gtctctgcgt atttctttct tatctagttt ccatggctac gtagataagt 4320

agcatggcgg gttaatcatt aactacagcc cgggcgttta aacagcgggc ggaggggtgg 4380

agtcgtgacg tgaattacgt catagggtta gggaggtcct gtattagagg tcacgtgagt 4440

gttttgcgac attttgcgac accatgtggt ctcgctgggg ggggggcccc gagtgagcac 4500

gcagggtctc cattttgaag cgggaggttt gaacgagcgc tggcgcgctc actggccgtc 4560

gttttacaac gtcgtgactg ggaaaaccct ggcgttaccc aacttaatcg ccttgcagca 4620

catccccctt tcgccagctg gcgtaatagc gaagaggccc gcaccgatcg cccttcccaa 4680

cagttgcgca gcctgaatgg cgaatggaaa ttgtaagcgt taatattttg ttaaaattcg 4740

cgttaaattt ttgttaaatc agctcatttt tttaaccaat aggccgaaat cggcaaaatc 4800

ccttataaat caaaagaata gaccgagata gggttgagtg ttgttccagt ttggaacaag 4860

agtccactat taagaacgtg gactccaacg tcaaagggcg aaaaaccgtc tatcagggcg 4920

atggcccact acgtgaacca tcaccctaat caagtttttt ggggtcgagg tgccgtaaag 4980

cactaaatcg gaaccctaaa gggagccccc gatttagagc ttgacgggga aagccggcga 5040

acgtggcgag aaaggaaggg aagaaagcga aaggagcggg cgctagggcg ctggcaagtg 5100

tagcggtcac gctgcgcgta accaccacac ccgccgcgct taatgcgccg ctacagggcg 5160

cgtcaggtgg cacttttcgg ggaaatgtgc gcggaacccc tatttgttta tttttctaaa 5220

tacattcaaa tatgtatccg ctcatgagac aataaccctg ataaatgctt caataatatt 5280

gaaaaaggaa gagtatgagt attcaacatt tccgtgtcgc ccttattccc ttttttgcgg 5340

cattttgcct tcctgttttt gctcacccag aaacgctggt gaaagtaaaa gatgctgaag 5400

atcagttggg tgcacgagtg ggttacatcg aactggatct caacagcggt aagatccttg 5460

agagttttcg ccccgaagaa cgttttccaa tgatgagcac ttttaaagtt ctgctatgtg 5520

gcgcggtatt atcccgtatt gacgccgggc aagagcaact cggtcgccgc atacactatt 5580

ctcagaatga cttggttgag tactcaccag tcacagaaaa gcatcttacg gatggcatga 5640

cagtaagaga attatgcagt gctgccataa ccatgagtga taacactgcg gccaacttac 5700

ttctgacaac gatcggagga ccgaaggagc taaccgcttt tttgcacaac atgggggatc 5760

atgtaactcg ccttgatcgt tgggaaccgg agctgaatga agccatacca aacgacgagc 5820

gtgacaccac gatgcctgta gcaatggcaa caacgttgcg caaactatta actggcgaac 5880

tacttactct agcttcccgg caacaattaa tagactggat ggaggcggat aaagttgcag 5940

gaccacttct gcgctcggcc cttccggctg gctggtttat tgctgataaa tctggagccg 6000

gtgagcgtgg gtctcgcggt atcattgcag cactggggcc agatggtaag ccctcccgta 6060

tcgtagttat ctacacgacg gggagtcagg caactatgga tgaacgaaat agacagatcg 6120

ctgagatagg tgcctcactg attaagcatt ggtaactgtc agaccaagtt tactcatata 6180

tactttagat tgatttaaaa cttcattttt aatttaaaag gatctaggtg aagatccttt 6240

ttgataatct catgaccaaa atcccttaac gtgagttttc gttccactga gcgtcagacc 6300

ccgtagaaaa gatcaaagga tcttcttgag atcctttttt tctgcgcgta atctgctgct 6360

tgcaaacaaa aaaaccaccg ctaccagcgg tggtttgttt gccggatcaa gagctaccaa 6420

ctctttttcc gaaggtaact ggcttcagca gagcgcagat accaaatact gttcttctag 6480

tgtagccgta gttaggccac cacttcaaga actctgtagc accgcctaca tacctcgctc 6540

tgctaatcct gttaccagtg gctgctgcca gtggcgataa gtcgtgtctt accgggttgg 6600

actcaagacg atagttaccg gataaggcgc agcggtcggg ctgaacgggg ggttcgtgca 6660

cacagcccag cttggagcga acgacctaca ccgaactgag atacctacag cgtgagctat 6720

gagaaagcgc cacgcttccc gaagggagaa aggcggacag gtatccggta agcggcaggg 6780

tcggaacagg agagcgcacg agggagcttc cagggggaaa cgcctggtat ctttatagtc 6840

ctgtcgggtt tcgccacctc tgacttgagc gtcgattttt gtgatgctcg tcaggggggc 6900

ggagcctatg gaaaaacgcc agcaacgcgg cctttttacg gttcctggcc ttttgctggc 6960

cttttgctca catgttcttt cctgcgttat cccctgattc tgtggataac cgtattaccg 7020

cctttgagtg agctgatacc gctcgccgca gccgaacgac cgagcgcagc gagtcagtga 7080

gcgaggaagc ggaagagcgc ccaatacgca aaccgcctct ccccgcgcgt tggccgattc 7140

attaatgcag ctggcacgac aggtttcccg actggaaagc gggcagtgag cgcaacgcaa 7200

ttaatgtgag ttagctcact cattaggcac cccaggcttt acactttatg cttccggctc 7260

gtatgttgtg tggaattgtg agcggataac aatttcacac aggaaacagc tatgaccatg 7320

attacgccaa 7330

<210> 11
<211> 5704
<212> DNA
<213> Artificial Sequence

<400> 11
cctgcaggca gctgcgcgct cgctcgctca ctgaggccgc ccgggcaaag cccgggcgtc 60

gggcgacctt tggtcgcccg gcctcagtga gcgagcgagc gcgcagagag ggagtggcca 120

actccatcac taggggttcc tgcggccgca cgcgtaagct ttgcaaagat ggataaagtt 180

ttaaacagag aggaatctct cgagccattg acgtcaataa tgacgtatgt tcccatagta 240

acgccaatag ggactttcca ttgacgtcaa tgggtggagt atttacggta aactgcccac 300

ttggcagtac atcaagtgta tcatatgcca agtacgcccc ctattgacgt caatgacggt 360

aaatggcccg cctggcatta tgcccagtac atgaccttat gggactttcc tacttggcag 420

tacatctacg tattagtcat cgctattacc atggtcgagg tgagccccac gttctgcttc 480

actctcccca tctccccccc ctccccaccc ccaattttgt atttatttat tttttaatta 540

ttttgtgcag cgatgggggc gggggggggg gggggggggc ggggcgaggc ggagaggtgc 600

ggcggcagcc aatcagagcg gcgcgctccg aaagtttcct tttatggcga ggcggcggcg 660

gcggcggccc tataaaaagc gaagcgcgcg gcgggcggga gtcgctgcgc gctgccttcg 720

ccccgtgccc cgctccgccg ccgcctcgcg ccgcccgccc cggctctgac tgaccgcgtt 780

actcccacag gtgagcgggc gggacggccc ttctcctccg ggctgtaatt agcgcttggt 840

ttaatgacgg cttgtttctt ttctgtggct gcgtgaaagc cttgaggggc tccgggaggg 900

ccctttgtgc ggggggagcg gctcggggct gtccgcgggg ggacggctgc cttcgggggg 960

gacggggcag ggcggggttc ggcttctggc gtgtgaccgg cggctctaga gcctctgcta 1020

accatgttca tgccttcttc tttttcctac agctcctggg caacgtgctg gttattgtgc 1080

tgtctcatca ttttggcaaa gaattggatc gaattcgcca ccatgggctc cggagccacc 1140

aacttttctc tgctgaagca ggctggcgac gtggaggaga acccaggacc tatggtgtct 1200

aagggcgagg aggataacat ggccagcctg cctgctacac acgagctgca catcttcgga 1260

agcatcaacg gcgtggactt tgatatggtg ggccagggaa ccggcaaccc aaacgacgga 1320

tacgaggagc tgaacctgaa gagcacaaag ggcgatctgc agttctcccc ctggattctg 1380

gtgcctcaca tcggatacgg ctttcaccag tacctgccat acccagacgg aatgagccca 1440

ttccaggctg ctatggtgga tggcagcggc taccaggtgc acaggaccat gcagtttgag 1500

gacggcgcct ctctgaccgt gaactacaga tacacatacg agggaagcca catcaagggc 1560

gaggcccagg tgaagggaac cggcttccct gctgacggac cagtgatgac caactccctg 1620

acagccgctg actggtgccg gtctaagaaa acatacccta acgataagac catcatctct 1680

acattcaagt ggagctacac cacaggaaac ggcaagaggt acagatccac cgcccggacc 1740

acatacacat ttgctaagcc aatggccgct aactacctga agaaccagcc catgtacgtg 1800

ttccgcaaga ccgagctgaa gcactccaag acagagctga acttcaagga gtggcagaag 1860

gcctttaccg acgtgatggg aatggatgag ctgtacaagg gatccgacta caaggaccac 1920

gatggcgact acaaggatca cgacatcgat tacaaggacg atgacgataa gaagcggaca 1980

gctgatggca gcgagttcga gtcccccaag aagaagagga aggtggagtg agctagcgat 2040

atcaagctta tcgataatca acctctggat tacaaaattt gtgaaagatt gactggtatt 2100

cttaactatg ttgctccttt tacgctatgt ggatacgctg ctttaatgcc tttgtatcat 2160

gctattgctt cccgtatggc tttcattttc tcctccttgt ataaatcctg gttgctgtct 2220

ctttatgagg agttgtggcc cgttgtcagg caacgtggcg tggtgtgcac tgtgtttgct 2280

gacgcaaccc ccactggttg gggcattgcc accacctgtc agctcctttc cgggactttc 2340

gctttccccc tccctattgc cacggcggaa ctcatcgccg cctgccttgc ccgctgctgg 2400

acaggggctc ggctgttggg cactgacaat tccgtggtgt tgtcggggaa atcatcgtcc 2460

tttccttggc tgctcgcctg tgttgccacc tggattctgc gcgggacgtc cttctgctac 2520

gtcccttcgg ccctcaatcc agcggacctt ccttcccgcg gcctgctgcc ggctctgcgg 2580

cctcttccgc gacttcgcct tcgccctcag acgagtcgga tctccctttg ggccgcctcc 2640

ccgcagatct aacttgttta ttgcagctta taatggttac aaataaagca atagcatcac 2700

aaatttcaca aataaagcat ttttttcact gcattctagt tgtggtttgt ccaaactcat 2760

caatgtatct tatcatgtct ggctagacac gtggccgcta ccccgaccac atgaagcagc 2820

acgacttctt caagtccgcc atgcccgaag gctacgtcca ggagcgcacc atcttcttca 2880

aggacgacgg caactacaag acccgcgccg aggtgaagtt cgagggcgac accctggtga 2940

accgcacgtg cggaccgagc ggccgcagga acccctagtg atggagttgg ccactccctc 3000

tctgcgcgct cgctcgctca ctgaggccgg gcgaccaaag gtcgcccgac gcccgggctt 3060

tgcccgggcg gcctcagtga gcgagcgagc gcgcagctgc ctgcaggggc gcctgatgcg 3120

gtattttctc cttacgcatc tgtgcggtat ttcacaccgc atacgtcaaa gcaaccatag 3180

tacgcgccct gtagcggcgc attaagcgcg gcgggtgtgg tggttacgcg cagcgtgacc 3240

gctacacttg ccagcgccct agcgcccgct cctttcgctt tcttcccttc ctttctcgcc 3300

acgttcgccg gctttccccg tcaagctcta aatcgggggc tccctttagg gttccgattt 3360

agtgctttac ggcacctcga ccccaaaaaa cttgatttgg gtgatggttc acgtagtggg 3420

ccatcgccct gatagacggt ttttcgccct ttgacgttgg agtccacgtt ctttaatagt 3480

ggactcttgt tccaaactgg aacaacactc aaccctatct cgggctattc ttttgattta 3540

taagggattt tgccgatttc ggcctattgg ttaaaaaatg agctgattta acaaaaattt 3600

aacgcgaatt ttaacaaaat attaacgttt acaattttat ggtgcactct cagtacaatc 3660

tgctctgatg ccgcatagtt aagccagccc cgacacccgc caacacccgc tgacgcgccc 3720

tgacgggctt gtctgctccc ggcatccgct tacagacaag ctgtgaccgt ctccgggagc 3780

tgcatgtgtc agaggttttc accgtcatca ccgaaacgcg cgagacgaaa gggcctcgtg 3840

atacgcctat ttttataggt taatgtcatg ataataatgg tttcttagac gtcaggtggc 3900

acttttcggg gaaatgtgcg cggaacccct atttgtttat ttttctaaat acattcaaat 3960

atgtatccgc tcatgagaca ataaccctga taaatgcttc aataatattg aaaaaggaag 4020

agtatgagta ttcaacattt ccgtgtcgcc cttattccct tttttgcggc attttgcctt 4080

cctgtttttg ctcacccaga aacgctggtg aaagtaaaag atgctgaaga tcagttgggt 4140

gcacgagtgg gttacatcga actggatctc aacagcggta agatccttga gagttttcgc 4200

cccgaagaac gttttccaat gatgagcact tttaaagttc tgctatgtgg cgcggtatta 4260

tcccgtattg acgccgggca agagcaactc ggtcgccgca tacactattc tcagaatgac 4320

ttggttgagt actcaccagt cacagaaaag catcttacgg atggcatgac agtaagagaa 4380

ttatgcagtg ctgccataac catgagtgat aacactgcgg ccaacttact tctgacaacg 4440

atcggaggac cgaaggagct aaccgctttt ttgcacaaca tgggggatca tgtaactcgc 4500

cttgatcgtt gggaaccgga gctgaatgaa gccataccaa acgacgagcg tgacaccacg 4560

atgcctgtag caatggcaac aacgttgcgc aaactattaa ctggcgaact acttactcta 4620

gcttcccggc aacaattaat agactggatg gaggcggata aagttgcagg accacttctg 4680

cgctcggccc ttccggctgg ctggtttatt gctgataaat ctggagccgg tgagcgtggg 4740

tctcgcggta tcattgcagc actggggcca gatggtaagc cctcccgtat cgtagttatc 4800

tacacgacgg ggagtcaggc aactatggat gaacgaaata gacagatcgc tgagataggt 4860

gcctcactga ttaagcattg gtaactgtca gaccaagttt actcatatat actttagatt 4920

gatttaaaac ttcattttta atttaaaagg atctaggtga agatcctttt tgataatctc 4980

atgaccaaaa tcccttaacg tgagttttcg ttccactgag cgtcagaccc cgtagaaaag 5040

atcaaaggat cttcttgaga tccttttttt ctgcgcgtaa tctgctgctt gcaaacaaaa 5100

aaaccaccgc taccagcggt ggtttgtttg ccggatcaag agctaccaac tctttttccg 5160

aaggtaactg gcttcagcag agcgcagata ccaaatactg tccttctagt gtagccgtag 5220

ttaggccacc acttcaagaa ctctgtagca ccgcctacat acctcgctct gctaatcctg 5280

ttaccagtgg ctgctgccag tggcgataag tcgtgtctta ccgggttgga ctcaagacga 5340

tagttaccgg ataaggcgca gcggtcgggc tgaacggggg gttcgtgcac acagcccagc 5400

ttggagcgaa cgacctacac cgaactgaga tacctacagc gtgagctatg agaaagcgcc 5460

acgcttcccg aagggagaaa ggcggacagg tatccggtaa gcggcagggt cggaacagga 5520

gagcgcacga gggagcttcc agggggaaac gcctggtatc tttatagtcc tgtcgggttt 5580

cgccacctct gacttgagcg tcgatttttg tgatgctcgt caggggggcg gagcctatgg 5640

aaaaacgcca gcaacgcggc ctttttacgg ttcctggcct tttgctggcc ttttgctcac 5700

atgt 5704

<210> 12
<211> 11635
<212> DNA
<213> Artificial Sequence

<400> 12
ggtacccaac tccatgctta acagtcccca ggtacagccc accctgcgtc gcaaccagga 60

acagctctac agcttcctgg agcgccactc gccctacttc cgcagccaca gtgcgcagat 120

taggagcgcc acttcttttt gtcacttgaa aaacatgtaa aaataatgta ctaggagaca 180

ctttcaataa aggcaaatgt ttttatttgt acactctcgg gtgattattt acccccaccc 240

cttgccgtct gcgccgttta aaaatcaaag gggttctgcc gcgcatcgct atgcgccact 300

ggcagggaca cgttgcgata ctggtgttta gtgctccact taaactcagg cacaaccatc 360

cgcggcagct cggtgaagtt ttcactccac aggctgcgca ccatcaccaa cgcgtttagc 420

aggtcgggcg ccgatatctt gaagtcgcag ttggggcctc cgccctgcgc gcgcgagttg 480

cgatacacag ggttgcagca ctggaacact atcagcgccg ggtggtgcac gctggccagc 540

acgctcttgt cggagatcag atccgcgtcc aggtcctccg cgttgctcag ggcgaacgga 600

gtcaactttg gtagctgcct tcccaaaaag ggtgcatgcc caggctttga gttgcactcg 660

caccgtagtg gcatcagaag gtgaccgtgc ccggtctggg cgttaggata cagcgcctgc 720

atgaaagcct tgatctgctt aaaagccacc tgagcctttg cgccttcaga gaagaacatg 780

ccgcaagact tgccggaaaa ctgattggcc ggacaggccg cgtcatgcac gcagcacctt 840

gcgtcggtgt tggagatctg caccacattt cggccccacc ggttcttcac gatcttggcc 900

ttgctagact gctccttcag cgcgcgctgc ccgttttcgc tcgtcacatc catttcaatc 960

acgtgctcct tatttatcat aatgctcccg tgtagacact taagctcgcc ttcgatctca 1020

gcgcagcggt gcagccacaa cgcgcagccc gtgggctcgt ggtgcttgta ggttacctct 1080

gcaaacgact gcaggtacgc ctgcaggaat cgccccatca tcgtcacaaa ggtcttgttg 1140

ctggtgaagg tcagctgcaa cccgcggtgc tcctcgttta gccaggtctt gcatacggcc 1200

gccagagctt ccacttggtc aggcagtagc ttgaagtttg cctttagatc gttatccacg 1260

tggtacttgt ccatcaacgc gcgcgcagcc tccatgccct tctcccacgc agacacgatc 1320

ggcaggctca gcgggtttat caccgtgctt tcactttccg cttcactgga ctcttccttt 1380

tcctcttgcg tccgcatacc ccgcgccact gggtcgtctt cattcagccg ccgcaccgtg 1440

cgcttacctc ccttgccgtg cttgattagc accggtgggt tgctgaaacc caccatttgt 1500

agcgccacat cttctctttc ttcctcgctg tccacgatca cctctgggga tggcgggcgc 1560

tcgggcttgg gagaggggcg cttctttttc tttttggacg caatggccaa atccgccgtc 1620

gaggtcgatg gccgcgggct gggtgtgcgc ggcaccagcg catcttgtga cgagtcttct 1680

tcgtcctcgg actcgagacg ccgcctcagc cgcttttttg ggggcgcgcg gggaggcggc 1740

ggcgacggcg acggggacga cacgtcctcc atggttggtg gacgtcgcgc cgcaccgcgt 1800

ccgcgctcgg gggtggtttc gcgctgctcc tcttcccgac tggccatttc cttctcctat 1860

aggcagaaaa agatcatgga gtcagtcgag aaggaggaca gcctaaccgc cccctttgag 1920

ttcgccacca ccgcctccac cgatgccgcc aacgcgccta ccaccttccc cgtcgaggca 1980

cccccgcttg aggaggagga agtgattatc gagcaggacc caggttttgt aagcgaagac 2040

gacgaggatc gctcagtacc aacagaggat aaaaagcaag accaggacga cgcagaggca 2100

aacgaggaac aagtcgggcg gggggaccaa aggcatggcg actacctaga tgtgggagac 2160

gacgtgctgt tgaagcatct gcagcgccag tgcgccatta tctgcgacgc gttgcaagag 2220

cgcagcgatg tgcccctcgc catagcggat gtcagccttg cctacgaacg ccacctgttc 2280

tcaccgcgcg taccccccaa acgccaagaa aacggcacat gcgagcccaa cccgcgcctc 2340

aacttctacc ccgtatttgc cgtgccagag gtgcttgcca cctatcacat ctttttccaa 2400

aactgcaaga tacccctatc ctgccgtgcc aaccgcagcc gagcggacaa gcagctggcc 2460

ttgcggcagg gcgctgtcat acctgatatc gcctcgctcg acgaagtgcc aaaaatcttt 2520

gagggtcttg gacgcgacga gaaacgcgcg gcaaacgctc tgcaacaaga aaacagcgaa 2580

aatgaaagtc actgtggagt gctggtggaa cttgagggtg acaacgcgcg cctagccgtg 2640

ctgaaacgca gcatcgaggt cacccacttt gcctacccgg cacttaacct accccccaag 2700

gttatgagca cagtcatgag cgagctgatc gtgcgccgtg cacgacccct ggagagggat 2760

gcaaacttgc aagaacaaac cgaggagggc ctacccgcag ttggcgatga gcagctggcg 2820

cgctggcttg agacgcgcga gcctgccgac ttggaggagc gacgcaagct aatgatggcc 2880

gcagtgcttg ttaccgtgga gcttgagtgc atgcagcggt tctttgctga cccggagatg 2940

cagcgcaagc tagaggaaac gttgcactac acctttcgcc agggctacgt gcgccaggcc 3000

tgcaaaattt ccaacgtgga gctctgcaac ctggtctcct accttggaat tttgcacgaa 3060

aaccgcctcg ggcaaaacgt gcttcattcc acgctcaagg gcgaggcgcg ccgcgactac 3120

gtccgcgact gcgtttactt atttctgtgc tacacctggc aaacggccat gggcgtgtgg 3180

cagcaatgcc tggaggagcg caacctaaag gagctgcaga agctgctaaa gcaaaacttg 3240

aaggacctat ggacggcctt caacgagcgc tccgtggccg cgcacctggc ggacattatc 3300

ttccccgaac gcctgcttaa aaccctgcaa cagggtctgc cagacttcac cagtcaaagc 3360

atgttgcaaa actttaggaa ctttatccta gagcgttcag gaattctgcc cgccacctgc 3420

tgtgcgcttc ctagcgactt tgtgcccatt aagtaccgtg aatgccctcc gccgctttgg 3480

ggtcactgct accttctgca gctagccaac taccttgcct accactccga catcatggaa 3540

gacgtgagcg gtgacggcct actggagtgt cactgtcgct gcaacctatg caccccgcac 3600

cgctccctgg tctgcaattc gcaactgctt agcgaaagtc aaattatcgg tacctttgag 3660

ctgcagggtc cctcgcctga cgaaaagtcc gcggctccgg ggttgaaact cactccgggg 3720

ctgtggacgt cggcttacct tcgcaaattt gtacctgagg actaccacgc ccacgagatt 3780

aggttctacg aagaccaatc ccgcccgcca aatgcggagc ttaccgcctg cgtcattacc 3840

cagggccaca tccttggcca attgcaagcc atcaacaaag cccgccaaga gtttctgcta 3900

cgaaagggac gggggtttta cctggacccc cagtccggcg aggagctcaa cccaatcccc 3960

ccgccgccgc agccctatca gcagccgcgg gcccttgctt cccaggatgg cacccaaaaa 4020

gaagctgcag ctgccgccgc cgccacccac ggacgaggag gaatactggg acagtcaggc 4080

agaggaggtt ttggacgagg aggaggagat gatggaagac tgggacagcc tagacgaagc 4140

ttccgaggcc gaagaggtgt cagacgaaac accgtcaccc tcggtcgcat tcccctcgcc 4200

ggcgccccag aaattggcaa ccgttcccag catcgctaca acctccgctc ctcaggcgcc 4260

gccggcactg cctgttcgcc gacccaaccg tagatgggac accactggaa ccagggccgg 4320

taagtctaag cagccgccgc cgttagccca agagcaacaa cagcgccaag gctaccgctc 4380

gtggcgcggg cacaagaacg ccatagttgc ttgcttgcaa gactgtgggg gcaacatctc 4440

cttcgcccgc cgctttcttc tctaccatca cggcgtggcc ttcccccgta acatcctgca 4500

ttactaccgt catctctaca gcccctactg caccggcggc agcggcagcg gcagcaacag 4560

cagcggtcac acagaagcaa aggcgaccgg atagcaagac tctgacaaag cccaagaaat 4620

ccacagcggc ggcagcagca ggaggaggag cgctgcgtct ggcgcccaac gaacccgtat 4680

cgacccgcga gcttagaaat aggatttttc ccactctgta tgctatattt caacaaagca 4740

ggggccaaga acaagagctg aaaataaaaa acaggtctct gcgctccctc acccgcagct 4800

gcctgtatca caaaagcgaa gatcagcttc ggcgcacgct ggaagacgcg gaggctctct 4860

tcagcaaata ctgcgcgctg actcttaagg actagtttcg cgccctttct caaatttaag 4920

cgcgaaaact acgtcatctc cagcggccac acccggcgcc agcacctgtc gtcagcgcca 4980

ttatgagcaa ggaaattccc acgccctaca tgtggagtta ccagccacaa atgggacttg 5040
cggctggagc tgcccaagac tactcaaccc gaataaacta catgagcgcg ggaccccaca 5100
tgatatcccg ggtcaacgga atccgcgccc accgaaaccg aattctcctc gaacaggcgg 5160
ctattaccac cacacctcgt aataacctta atccccgtag ttggcccgct gccctggtgt 5220
accaggaaag tcccgctccc accactgtgg tacttcccag agacgcccag gccgaagttc 5280
agatgactaa ctcaggggcg cagcttgcgg gcggctttcg tcacagggtg cggtcgcccg 5340
ggcgttttag ggcggagtaa cttgcatgta ttgggaattg tagttttttt aaaatgggaa 5400
gtgacgtatc gtgggaaaac ggaagtgaag atttgaggaa gttgtgggtt ttttggcttt 5460
cgtttctggg cgtaggttcg cgtgcggttt tctgggtgtt ttttgtggac tttaaccgtt 5520
acgtcatttt ttagtcctat atatactcgc tctgtacttg gcccttttta cactgtgact 5580
gattgagctg gtgccgtgtc gagtggtgtt ttttaatagg tttttttact ggtaaggctg 5640
actgttatgg ctgccgctgt ggaagcgctg tatgttgttc tggagcggga gggtgctatt 5700
ttgcctaggc aggagggttt ttcaggtgtt tatgtgtttt tctctcctat taattttgtt 5760
atacctccta tgggggctgt aatgttgtct ctacgcctgc gggtatgtat tcccccgggc 5820
tatttcggtc gctttttagc actgaccgat gttaaccaac ctgatgtgtt taccgagtct 5880
tacattatga ctccggacat gaccgaggaa ctgtcggtgg tgctttttaa tcacggtgac 5940
cagttttttt acggtcacgc cggcatggcc gtagtccgtc ttatgcttat aagggttgtt 6000
tttcctgttg taagacaggc ttctaatgtt taaatgtttt tttttttgtt attttatttt 6060
gtgtttaatg caggaacccg cagacatgtt tgagagaaaa atggtgtctt tttctgtggt 6120
ggttccggaa cttacctgcc tttatctgca tgagcatgac tacgatgtgc ttgctttttt 6180
gcgcgaggct ttgcctgatt ttttgagcag caccttgcat tttatatcgc cgcccatgca 6240
acaagcttac ataggggcta cgctggttag catagctccg agtatgcgtg tcataatcag 6300
tgtgggttct tttgtcatgg ttcctggcgg ggaagtggcc gcgctggtcc gtgcagacct 6360
gcacgattat gttcagctgg ccctgcgaag ggacctacgg gatcgcggta tttttgttaa 6420
tgttccgctt ttgaatctta tacaggtctg tgaggaacct gaatttttgc aatcatgatt 6480
cgctgcttga ggctgaaggt ggagggcgct ctggagcaga tttttacaat ggccggactt 6540
aatattcggg atttgcttag agacatattg ataaggtggc gagatgaaaa ttatttgggc 6600
atggttgaag gtgctggaat gtttatagag gagattcacc ctgaagggtt tagcctttac 6660
gtccacttgg acgtgagggc agtttgcctt ttggaagcca ttgtgcaaca tcttacaaat 6720
gccattatct gttctttggc tgtagagttt gaccacgcca ccggagggga gcgcgttcac 6780
ttaatagatc ttcattttga ggttttggat aatcttttgg aataaaaaaa aaaaaacatg 6840

gttcttccag ctcttcccgc tcctcccgtg tgtgactcgc agaacgaatg tgtaggttgg 6900

ctgggtgtgg cttattctgc ggtggtggat gttatcaggg cagcggcgca tgaaggagtt 6960

tacatagaac ccgaagccag ggggcgcctg gatgctttga gagagtggat atactacaac 7020

tactacacag agcgagctaa gcgacgagac cggagacgca gatctgtttg tcacgcccgc 7080

acctggtttt gcttcaggaa atatgactac gtccggcgtt ccatttggca tgacactacg 7140

accaacacga tctcggttgt ctcggcgcac tccgtacagt agggatcgcc tacctccttt 7200

tgagacagag acccgcgcta ccatactgga ggatcatccg ctgctgcccg aatgtaacac 7260

tttgacaatg cacaacgtga gttacgtgcg aggtcttccc tgcagtgtgg gatttacgct 7320

gattcaggaa tgggttgttc cctgggatat ggttctgacg cgggaggagc ttgtaatcct 7380

gaggaagtgt atgcacgtgt gcctgtgttg tgccaacatt gatatcatga cgagcatgat 7440

gatccatggt tacgagtcct gggctctcca ctgtcattgt tccagtcccg gttccctgca 7500

gtgcatagcc ggcgggcagg ttttggccag ctggtttagg atggtggtgg atggcgccat 7560

gtttaatcag aggtttatat ggtaccggga ggtggtgaat tacaacatgc caaaagaggt 7620

aatgtttatg tccagcgtgt ttatgagggg tcgccactta atctacctgc gcttgtggta 7680

tgatggccac gtgggttctg tggtccccgc catgagcttt ggatacagcg ccttgcactg 7740

tgggattttg aacaatattg tggtgctgtg ctgcagttac tgtgctgatt taagtgagat 7800

cagggtgcgc tgctgtgccc ggaggacaag gcgtctcatg ctgcgggcgg tgcgaatcat 7860

cgctgaggag accactgcca tgttgtattc ctgcaggacg gagcggcggc ggcagcagtt 7920

tattcgcgcg ctgctgcagc accaccgccc tatcctgatg cacgattatg actctacccc 7980

catgtaggcg tggacttccc cttcgccgcc cgttgagcaa ccgcaagttg gacagcagcc 8040

tgtggctcag cagctggaca gcgacatgaa cttaagcgag ctgcccgggg agtttattaa 8100

tatcactgat gagcgtttgg ctcgacagga aaccgtgtgg aatataacac ctaagaatat 8160

gtctgttacc catgatatga tgctttttaa ggccagccgg ggagaaagga ctgtgtactc 8220

tgtgtgttgg gagggaggtg gcaggttgaa tactagggtt ctgtgagttt gattaaggta 8280

cggtgatcaa tataagctat gtggtggtgg ggctatacta ctgaatgaaa aatgacttga 8340

aattttctgc aattgaaaaa taaacacgtt gaaacataac atgcaacagg ttcacgattc 8400

tttattcctg ggcaatgtag gagaaggtgt aagagttggt agcaaaagtt tcagtggtgt 8460

attttccact ttcccaggac catgtaaaag acatagagta agtgcttacc tcgctagttt 8520

ctgtggattc actagaatcg atgtaggatg ttgcccctcc tgacgcggta ggagaagggg 8580

agggtgccct gcatgtctgc cgctgctctt gctcttgccg ctgctgagga ggggggcgca 8640

tctgccgcag caccggatgc atctgggaaa agcaaaaaag gggctcgtcc ctgtttccgg 8700

aggaatttgc aagcggggtc ttgcatgacg gggaggcaaa cccccgttcg ccgcagtccg 8760

gccggcccga gactcgaacc gggggtcctg cgactcaacc cttggaaaat aaccctccgg 8820

ctacagggag cgagccactt aatgctttcg ctttccagcc taaccgctta cgccgcgcgc 8880

ggccagtggc caaaaaagct agcgcagcag ccgccgcgcc tggaaggaag ccaaaaggag 8940

cgctcccccg ttgtctgacg tcgcacacct gggttcgaca cgcgggcggt aaccgcatgg 9000

atcacggcgg acggccggat ccggggttcg aaccccggtc gtccgccatg atacccttgc 9060

gaatttatcc accagaccac ggaagagtgc ccgcttacag gctctccttt tgcacggtct 9120

agagcgtcaa cgactgcgca cgcctcaccg gccagagcgt cccgaccatg gagcactttt 9180

tgccgctgcg caacatctgg aaccgcgtcc gcgactttcc gcgcgcctcc accaccgccg 9240

ccggcatcac ctggatgtcc aggtacatct acggattacg tcgacgttta aaccatatga 9300

tcagctcact caaaggcggt aatacggtta tccacagaat caggggataa cgcaggaaag 9360

aacatgtgag caaaaggcca gcaaaaggcc aggaaccgta aaaaggccgc gttgctggcg 9420

tttttccata ggctccgccc ccctgacgag catcacaaaa atcgacgctc aagtcagagg 9480

tggcgaaacc cgacaggact ataaagatac caggcgtttc cccctggaag ctccctcgtg 9540

cgctctcctg ttccgaccct gccgcttacc ggatacctgt ccgcctttct cccttcggga 9600

agcgtggcgc tttctcatag ctcacgctgt aggtatctca gttcggtgta ggtcgttcgc 9660

tccaagctgg gctgtgtgca cgaacccccc gttcagcccg accgctgcgc cttatccggt 9720

aactatcgtc ttgagtccaa cccggtaaga cacgacttat cgccactggc agcagccact 9780

ggtaacagga ttagcagagc gaggtatgta ggcggtgcta cagagttctt gaagtggtgg 9840

cctaactacg gctacactag aagaacagta tttggtatct gcgctctgct gaagccagtt 9900

accttcggaa aaagagttgg tagctcttga tccggcaaac aaaccaccgc tggtagcggt 9960

ggtttttttg tttgcaagca gcagattacg cgcagaaaaa aaggatctca agaagatcct 10020

ttgatctttt ctacggggtc tgacgctcag tggaacgaaa actcacgtta agggattttg 10080

gtcatgagat tatcaaaaag gatcttcacc tagatccttt taaattaaaa atgaagtttt 10140

aaatcaatct aaagtatata tgagtaaact tggtctgaca gttaccaatg cttaatcagt 10200

gaggcaccta tctcagcgat ctgtctattt cgttcatcca tagttgcctg actccccgtc 10260

gtgtagataa ctacgatacg ggagggctta ccatctggcc ccagtgctgc aatgataccg 10320

cgagacccac gctcaccggc tccagattta tcagcaataa accagccagc cggaagggcc 10380

gagcgcagaa gtggtcctgc aactttatcc gcctccatcc agtctattaa ttgttgccgg 10440

gaagctagag taagtagttc gccagttaat agtttgcgca acgttgttgc cattgctaca 10500

ggcatcgtgg tgtcacgctc gtcgtttggt atggcttcat tcagctccgg ttcccaacga 10560

tcaaggcgag ttacatgatc ccccatgttg tgcaaaaaag cggttagctc cttcggtcct 10620

ccgatcgttg tcagaagtaa gttggccgca gtgttatcac tcatggttat ggcagcactg 10680

cataattctc ttactgtcat gccatccgta agatgctttt ctgtgactgg tgagtactca 10740

accaagtcat tctgagaata gtgtatgcgg cgaccgagtt gctcttgccc ggcgtcaata 10800

cgggataata ccgcgccaca tagcagaact ttaaaagtgc tcatcattgg aaaacgttct 10860

tcggggcgaa aactctcaag gatcttaccg ctgttgagat ccagttcgat gtaacccact 10920

cgtgcaccca actgatcttc agcatctttt actttcacca gcgtttctgg gtgagcaaaa 10980

acaggaaggc aaaatgccgc aaaaaaggga ataagggcga cacggaaatg ttgaatactc 11040

atactcttcc tttttcaata ttattgaagc atttatcagg gttattgtct catgagcgga 11100

tacatatttg aatgtattta gaaaaataaa caaatagggg ttccgcgcac atttccccga 11160

aaagtgccac ctaaattgta agcgttaata ttttgttaaa attcgcgtta aatttttgtt 11220

aaatcagctc attttttaac caataggccg aaatcggcaa aatcccttat aaatcaaaag 11280

aatagaccga gatagggttg agtgttgttc cagtttggaa caagagtcca ctattaaaga 11340

acgtggactc caacgtcaaa gggcgaaaaa ccgtctatca gggcgatggc ccactacgtg 11400

aaccatcacc ctaatcaagt tttttggggt cgaggtgccg taaagcacta aatcggaacc 11460

ctaaagggag cccccgattt agagcttgac ggggaaagcc ggcgaacgtg gcgagaaagg 11520

aagggaagaa agcgaaagga gcgggcgcta gggcgctggc aagtgtagcg gtcacgctgc 11580

gcgtaaccac cacacccgcc gcgcttaatg cgccgctaca gggcgcgatg gatcc 11635

## Description

### Technical Field

The present disclosure relates to the field of biotechnology, in particular, to an adeno-associated virus having a variant capsid protein and use thereof.

### Background

Hearing loss is one of the most common sensory disabilities, affecting more than 6.8% of the world's population (about 500 million people). The most widespread treatment for hearing loss is the use of the hearing device, which amplifies sound, enhances sound delivery, or directly stimulates neurons. This method is currently the best choice for treating hearing loss, but unfortunately, in noisy environments, the hearing sensitivity and perception of natural sounds are still limited. Therefore, better alternative strategies are needed to treat hearing loss. In recent years, gene therapy has become a promising strategy for the treatment of genetic diseases.

Half of the cases of sensorineural hearing loss are caused by genetic mutations in hair cells and supporting cells. Gene mutations in spiral ganglion neurons may also cause some hearing impairments. More than 100 mutations in deafness genes have been discovered. Hereditary hearing loss is a typical single-source disease, and a single mutation may cause hearing loss. Therefore, gene therapy is an ideal and promising potential treatment strategy that can maintain or reconstruct hearing function. For hereditary hearing loss, most deafness genes are expressed in hair cells, and mutations in some genes may affect spiral ganglion neurons. A large number of gene mutations directly affect the various functions of hair cells. However, some key deafness genes such as GJB2 are mainly expressed in supporting cells, and their mutations would affect the functions of the supporting cells, which would be accompanied by the damage to the hair cells and eventually lead to hearing loss. According to reports, there are more than 300 mutations in GJB2 that can cause genetic hearing loss. Mutations in GJB2 are the most common cause of hereditary hearing loss in humans, accounting for approximately 50% of autosomal recessive hearing loss and 15-18% of all hereditary hearing loss. Therefore, gene therapy for hereditary hearing loss needs to target both hair cells and supporting cells.

Adenovirus associated virus (AAV) is a non-enveloped icosahedral virus with a diameter of 18-26 nm. The capsid structure of AAV is composed of three capsid proteins (VP1, VP2, and VP3). The capsid contains a 4.7kb single-stranded DNA (ssDNA), carrying two genes rep and cap, flanked by inverted terminal repeats (ITRs). Both rep and cap have multiple open reading frames (ORFs), which express the proteins needed for genome replication and packaging. Since the ITRs sequence may be directly applied to ordinary plasmids, the foreign DNA may be directly inserted into the ITRs to form a recombinant plasmid. The AAV virus gene can be cloned into a second plasmid and packaged to form a new recombinant AAV virus under the action of helper plasmid Helper. Currently, the commonly used AAVs include AAV-1, AAV-2, AAV-5, AAV-8, AAV-9 and AAV-DJ.

Several viral and non-viral gene transfer strategies have been tested in gene therapy. Among these methods, AAV-mediated gene therapy has progressed rapidly in the past decade, and more than 170 human trials (7.2%) have used AAV vectors. AAV vector has excellent safety, low immunogenicity and high transfection efficiency. The US FDA approved the first AAV-mediated gene therapy in 2017 for the treatment of rare hereditary eye diseases. However, to date, there is no gene therapy for cochlea based on AAV vectors. This may be caused by the lack of AAV with high conduction efficiency in different cell types in the cochlea. Several studies have tested AAV-mediated gene therapy for cochlea in animal models and showed promising results. However, most studies focus on hair cells and spiral ganglion neurons, due to the lack of a safe and effective AAV targeting the supporting cells. Some AAV serotypes have been evaluated in the cochlea, and all tested AAVs have a very low infection rate to the supporting cells, less than 13%. Therefore, it is necessary to design a new AAV that can effectively transfect supporting cells for the further application of gene therapy for hereditary hearing loss.

### Summary

The present disclosure provides an adeno-associated virus having a variant capsid protein and use thereof, to solve the problem that the AAV in the traditional technology has a low infection rate to hair cells and supporting cells.

Based on inserting an amino acid sequence shown in SEQ ID NO.1 between the N589 and R590 of the capsid protein VP1 of the wild-type AAV-DJ, the obtained variant AAV-ie has stronger infectivity to hair cells and supporting cells.

In one aspect, the present disclosure provides a capsid protein VP1 of a variant adeno-associated virus AAV-ie. The capsid protein VP1 of the variant adeno-associated virus AAV-ie inserts an amino acid fragment between N589 and R590 of the capsid protein VP1 of the wild-type AAV-DJ, and the amino acid sequence of the amino acid fragment is shown in SEQ ID NO.1, specifically: DGTLAVPFK (SEQ ID NO. 1)

In some embodiments of the present disclosure, the amino acid sequence of the capsid protein VP1 of the wild-type AAV-DJ is shown in SEQ ID NO. 3, specifically:

MAADGYLPDWLEDTLSEGIRQWWKLKPGPPPPKPAERHKDDSRGLVLPGYK
YLGPFNGLDKGEPVNEADAAALEHDKAYDRQLDSGDNPYLKYNHADAEFQ
ERLKEDTSFGGNLGRAVFQAKKRLLEPLGLVEEAAKTAPGKKRPVEHSPVEP
DSSSGTGKAGQQPARKRLNFGQTGDADSVPDPQPIGEPPAAPSGVGSLTMAA
GGGAPMADNNEGADGVGNSSGNWHCDSTWMGDRVITTSTRTWALPTYNN
HLYKQISNSTSGGSSNDNAYFGYSTPWGYFDFNRFHCHFSPRDWQRLINNN

WGFRPKRLSFKLFNIQVKEVTQNEGTKTIANNLTSTIQVFTDSEYQLPYVLGS
AHQGCLPPFPADVFMIPQYGYLTLNNGSQAVGRSSFYCLEYFPSQMLRTGNN
FQFTYTFEDVPFHSSYAHSQSLDRLMNPLIDQYLYYLSRTQTTGGTTNTQTLG
FSQGGPNTMANQAKNWLPGPCYRQQRVSKTSADNNNSEYSWTGATKYHLN
GRDSLVNPGPAMASHKDDEEKFFPQSGVLIFGKQGSEKTNVDIEKVMITDEE
EIRTTNPVATEQYGSVSTNLQRGNRQAATADVNTQGVLPGMVWQDRDVYLQ
GPIWAKIPHTDGHFHPSPLMGGFGLKHPPPQILIKNTPVPADPPTTFNQSKLNS
FITQYSTGQVSVEIEWELQKENSKRWNPEIQYTSNYYKSTSVDFAVNTEGVYS
EPRPIGTRYLTRNL (SEQ ID NO. 3)

In some embodiments of the present disclosure, the amino acid sequence of the capsid protein VP1 of the variant adeno-associated virus AAV-ie is shown in SEQ ID NO. 4, specifically:

MAADGYLPDWLEDTLSEGIRQWWKLKPGPPPPKPAERHKDDSRGLVLPGYK
YLGPFNGLDKGEPVNEADAAALEHDKAYDRQLDSGDNPYLKYNHADAEFQ
ERLKEDTSFGGNLGRAVFQAKKRLLEPLGLVEEAAKTAPGKKRPVEHSPVEP
DSSSGTGKAGQQPARKRLNFGQTGDADSVPDPQPIGEPPAAPSGVGSLTMAA
GGGAPMADNNEGADGVGNSSGNWHCDSTWMGDRVITTSTRTWALPTYNN
HLYKQISNSTSGGSSNDNAYFGYSTPWGYFDFNRFHCHFSPRDWQRLINNN
WGFRPKRLSFKLFNIQVKEVTQNEGTKTIANNLTSTIQVFTDSEYQLPYVLGS
AHQGCLPPFPADVFMIPQYGYLTLNNGSQAVGRSSFYCLEYFPSQMLRTGNN
FQFTYTFEDVPFHSSYAHSQSLDRLMNPLIDQYLYYLSRTQTTGGTTNTQTLG
FSQGGPNTMANQAKNWLPGPCYRQQRVSKTSADNNNSEYSWTGATKYHLN
GRDSLVNPGPAMASHKDDEEKFFPQSGVLIFGKQGSEKTNVDIEKVMITDEE
EIRTTNPVATEQYGSVSTNLQRGNDGTLAVPFKRQAATADVNTQGVLPGMV
WQDRDVYLQGPIWAKIPHTDGHFHPSPLMGGFGLKHPPPQILIKNTPVPADPP
TTFNQSKLNSFITQYSTGQVSVEIEWELQKENSKRWNPEIQYTSNYYKSTSVD
FAVNTEGVYSEPRPIGTRYLTRNL (SEQ ID NO. 4)

In another aspect, the present disclosure provides an isolated nucleic acid containing the nucleotide sequence encoding the above-mentioned capsid protein VP1 of the variant adeno-associated virus AAV-ie.

In another aspect, the present disclosure provides a construct containing the above-mentioned isolated nucleic acid. The construct may generally be obtained by inserting the above-mentioned isolated nucleic acid into a suitable expression vector. Those skilled in the art may select a suitable expression vector. For example, the expression vector may be Rep2-Capsid and the like.

In another aspect, the present disclosure provides a host cell, containing the above-mentioned construct or incorporating the above-mentioned exogenous isolated nucleic acid in the genome, or containing the above-mentioned variant adeno-associated virus AAV-ie. The host cell may be a eukaryotic cell and/or a prokaryotic cell, specifically, a mouse cell, a human cell, etc., more specifically, a human embryonic kidney cell, etc., and more specifically, a HEK293FT cell.

In another aspect, the present disclosure provides a variant adeno-associated virus AAV-ie, including the above-mentioned capsid protein VP1 of the variant adeno-associated virus AAV-ie. That is, an amino acid fragment as shown in SEQ ID NO.1 is inserted between N589 and R590 of the capsid protein VP1 of the wild-type AAV-DJ.

Further, the variant adeno-associated virus AAV-ie further includes a heterologous nucleotide sequence encoding a target product. The heterologous nucleotide sequence encoding the target product may be carried by various capsid proteins. The heterologous nucleotide sequence encoding the target product may generally be a construct, which may generally contain a nucleic acid encoding the target product. The construct may generally be obtained by inserting the nucleic acid encoding the target product into a suitable expression vector. Those skilled in the art may select a suitable expression vector. For example, the above expression vector may include, but not limited to, pAAV-CAG, pAAV-TRE, pAAV-EF1a, pAAV-GFAP promoter, pAAV-Lgr5 promoter, pAAV-Sox2 promoter and the like.

Further, the target product is a nucleic acid or a protein, and the nucleic acid may be small guide RNA (sgRNA), interfering RNA (RNAi), etc.

The variant adeno-associated virus AAV-ie may serve as a carrier material to introduce exogenous genes into the cells of the subject. Compared with the parental wild-type AAV-DJ, the infection rate of the variant adeno-associated virus AAV-ie to hair cells and supporting cells has significantly increased.

In another aspect, the present disclosure provides a pharmaceutical composition, including the variant adeno-associated virus AAV-ie as described above and pharmaceutically acceptable excipients.

In another aspect, the present disclosure provides the use of the variant adeno-associated virus AAV-ie for delivering a target product to hair cells and/or supporting cells of an individual. The delivery of the target product may be for non-diagnostic/therapeutic purposes, for example, it may be in vitro to deliver the target product to the isolated hair cells and/or supporting cells. The hair cells generally include outer hair cells and/or inner hair cells.

Further, the target product is a nucleic acid or a protein, and the nucleic acid may be small guide RNA (sgRNA), interfering RNA (RNAi), etc.

In another aspect, the present disclosure provides the use of the variant adeno-associated virus AAV-ie in the preparation of drugs for the treatment of a hearing impairment disease caused by cochlear injury in an individual.

Further, the hearing impairment disease is a disease related to hair cells and/or supporting cells and/or spiral neuron cells.

Further, the hearing impairment disease is a disease related to genetic defects, environmental damage or aging, for example, a related disease caused by gene mutation, for another example, a related disease caused by noise or drugs, for yet another example, a related disease caused by aging.

Further, the hearing impairment disease may be a disease related to cell damage, specifically cochlear hair cell damage and supporting cell damage, and more specifically, cochlear hair cell damage caused by gene mutation, supporting cell damage caused by gene mutation, cell damage caused by noise, cell damage caused by drugs, or aging.

Further, the variant adeno-associated virus AAV-ie serves as a carrier for delivering the target product. As described above, the adeno-associated virus having a variant capsid protein and use thereof of the present disclosure have the following beneficial effects:

The variant adeno-associated virus AAV-ie has higher infectivity to hair cells and supporting cells, can efficiently infect hair cells and supporting cells, and can also efficiently infect spiral ganglion neurons, which is greatly improved compared with the parents, therefore provides better technical support for scientific research.

### Brief Description of the Drawings

FIG. 1a shows the expression of green fluorescent protein mNeonGreen after the wild-type AAV-DJ infects HEK 293T cells in Embodiment 1.
FIG. 1b shows the expression of green fluorescent protein mNeonGreen after the variant AAV-ie infects HEK 293T cells in Embodiment 1.
FIG. 1c shows the statistics of the fluorescence ratio of HEK 293T cells infected by wild-type AAV-DJ and variant AAV-ie in Embodiment 1.
FIG. 2a shows the immunostaining photographs of green fluorescence mNeonGreen and supporting cell specific marker protein Sox2 after the AAV1, AAV6, AAV9, AAV-DJ, AAV-ie and Anc80L65 infects the in-vitro cultured cochlear tissue in vitro in Embodiment 2.
FIG. 2b shows the statistical results of the data in FIG. 2a.
FIG. 3a shows the immunostaining photographs of green fluorescence mNeonGreen and hair cell specific marker protein Myo7a after the AAV1, AAV6, AAV9, AAV-DJ, AAV-ie and Anc80L65 infects the in-vitro cultured cochlear tissue in vitro in Embodiment 2.
FIG. 3b shows the statistical results of the data in FIG. 3a.
FIG. 4a shows the expression of green fluorescence mNeonGreen and the staining results of the supporting cell marker protein Sox2 represented by magenta in three areas of the cochlea in the large field of view in Embodiment 3: apex, middle and base. 14 days after the AAV-ie virus is injected into the round window of newborn mice, the cochlea is dissected out and immunostained by supporting cell specific marker protein Sox2, and then the results of green fluorescence mNeonGreen and immunostaining are photographed.
FIG. 4b shows the immunostaining photographs of green fluorescence mNeonGreen and Sox2-positive supporting cell after the AAV1, AAV6, AAV8, AAV9, DJ8, Anc80L65, AAV-DJ and AAV-ie infects the cochlear tissue in Embodiment 3.
FIG. 4c shows the statistical results of the data in FIG. 4b.
FIG. 5a shows the expression of green fluorescence mNeonGreen and the staining results of the hair cell marker protein Myo7a represented by magenta in AAV-DJ and AAV-ie in Embodiment 3.
FIG. 5b shows the statistical results of the data in FIG. 5a.
FIG. 6a shows the expression of green fluorescence mNeonGreen and the staining results of the spiral ganglion neuron marker protein NeurN represented by magenta in three areas: apex, middle and base.
FIG. 6b shows the statistical results in FIG. 6a.
FIG. 7 shows the immunostaining results of mouse cochlear samples in Embodiment 4.
FIG. 8a shows the immunostaining results of mouse utricle samples in Embodiment 5.
FIG. 8b shows the immunostaining results of human utricle samples in Embodiment 5.
FIG. 8c is an enlarged view of FIG. 8b.

### Detailed Description of the Preferred Embodiments

The embodiments of the present disclosure will be described below through exemplary embodiments. Those skilled in the art can easily understand other advantages and effects of the present disclosure according to contents disclosed by the specification. The present disclosure can also be implemented or applied through other different exemplary embodiments. Various modifications or changes can also be made to all details in the specification based on different points of view and applications without departing from the spirit of the present disclosure.

Before further describing the specific embodiments of the present disclosure, it is understood that the scope of the present disclosure is not limited to the specific embodiments described below; It is also to be understood that the terminology of the disclosure is used to describe the specific embodiments, and not to limit the scope of the disclosure; In the present specification and claims, the singular forms "a", "an" and "the" include the plural forms, unless specifically stated otherwise.

When the numerical values are given by the embodiments, it is to be understood that the two endpoints of each numerical range and any one between the two may be selected unless otherwise stated. Unless otherwise defined, all technical and scientific terms used in the present disclosure have the same meaning as commonly understood by one skill in the art. In addition to the specific method, equipment and material used in the embodiments, any method, equipment and material in the existing technology similar or equivalent to the method, equipment and material mentioned in the embodiments of the present disclosure may be used to realize the invention according to the grasp of the existing technology and the record of the invention by those skilled in the art.

Unless otherwise stated, the experimental methods, detection methods, and preparation methods disclosed in the present invention all employ conventional techniques of molecular biology, biochemistry, chromatin structure and analysis, analytical chemistry, cell culture, recombinant DNA technology in the technical field and related fields. These techniques are well described in the prior literature. For details, see Sambrook et al. MOLECULAR CLONING: A LABORATORY MANUAL, Second edition, Cold Spring Harbor Laboratory Press, 1989 and Third edition, 2001; Ausubel et al, CURRENT PROTOCOLS IN MOLECULAR BIOLOGY, John Wiley & Sons, New York, 1987 and periodic updates; The series METHODS IN ENZYMOLOGY, Academic Press, San Diego; Wolffe, CHROMATIN STRUCTURE AND FUNCTION, Third edition, Academic Press, San Diego, 1998; METHODS IN ENZYMOLOGY, Vol. 304, Chromatin (P.M. Wassarman and A.P. Wolffe, eds.), Academic Press, San Diego, 1999; and METHODS IN MOLECULAR BIOLOGY, Vol. 119, Chromatin Protocols (P. B. Becker, ed.) Humana Press, Totowa, 1999, and the like.

### Materials and sources:

Young mice: Shanghai Lingchang Biological Technology Co., Ltd.

Various AAVs: synthesized and constructed by Nanjing Genscript Co., Ltd.

Donkey serum: Shanghai Yeasen Biotechnology Co., Ltd.

Antibodies and dilution ratio of the antibodies when staining:

Primary antibody: myosin 7A (Myo7a, # 25-6790 Proteus Biosciences, 1: 1000), Sox2 (Sox-2, # sc-17320, Santa Cruz Biotechnology, 1: 1000), Flag (Flag, # F3165, Sigma Aldrich, 1: 1000), NeuN (NeuN, # 12943S, Cell Signaling Technology, 1: 500).

Secondary antibody: the secondary antibodies of anti-rat, mouse, rabbit and goat labeled with three different colors (TRIC, FITC, Cy5) were from Invitrogen.

Reagents for cell and tissue culture: DMEM (Hyclone), fetal bovine serum (Lensa), supplement N2 (ThermoFisher), ampicillin (ThermoFisher), double antibody (ThermoFisher).

Consumables for cell and tissue culture: commonly used consumables including various culture dishes, centrifuge tubes, pipettes and disposable filters were purchased from Corning.

### Embodiment 1 Construction of AAV variants and infection of HEK 293T cells

### 1. Construction of AAV variant (named AAV-ie) Rep-Cap plasmid

AAV packaging requires three plasmids: genomic plasmid containing the target gene, Rep-Cap plasmid and Helper plasmid.

The sequence of the Cap protein in the Rep-Cap plasmid determines the different serotypes of AAV, which in turn affects the preference of AAV to infect cells. Therefore, a new AAV type can be obtained by modifying the Cap protein.

### (1) Construction, enzyme digestion and purification of vector

The Rep-Cap plasmid of the parental AAV-DJ was synthesized by Nanjing Genscript Co., Ltd. (www.genscript.com.cn).

First, a unique NheI restriction enzyme site was introduced between amino acids 589 and 590 of the VP1 capsid protein of the wild-type AAV-DJ by polymerase chain reaction (PCR) mutagenesis, the primer was synthesized by Nanjing Genscript Co., Ltd. (www.genscript.com.cn). Then the obtained VP1 capsid protein was digested with restriction enzyme NheI and recovered (Axygen: AP-GX-250G). The recovered fragments were tested for concentration by Nanodrop 2000.

The DNA sequence (SEQ ID NO. 2: gatgggactttggcggtgccttttaag) encoding DGTLAVPFK was synthesized by Nanjing Genscript Co., Ltd. (www.genscript.com.cn). The synthesized fragments were dissolved to 10 µM with ultrapure water.

### (2) Vector connection, transformation and plasmid extraction

The recovered backbone vector and the DNA sequence fragment encoding DGTLAVPFK were recombined and connected (Novoprotein: NR001A), the connection system is as follows: recombinant connection buffer 2 µL, backbone vector 30 ng, DNA fragment encoding DGTLAVPFK 1 µL, recombinant ligase 0.5 µL, filling ddH₂O to 10 µL, reacting at 50 °C for 20 minutes.

The transformation steps are as follows: taking 100 µL competent cells (TransGen: CD201), thawing on ice; mixing 10 µL of ligation product with the competent cells, then placing on ice for 20 minutes; heat shocking at 42 °C for 60 seconds; placing on ice for 2 minutes, adding 400 µL of resuscitation LB medium (MDBio: L001-1kg), placing on a shaker for 30 minutes; taking 70µL of the shaken medium, spreading on an ampicillin plate (50 µg/ml, 37 °C incubator, and incubating for 14 hours).

Selecting the monoclonal bacteria, expanding the culture in 4ml liquid LB medium, and extracting the plasmid after 14 hours (Axygene: AP-MN-P-250G).

The steps are as follows: centrifuging the bacterial solution at 4000 rpm for 10 minutes, and discarding the supernatant medium; adding 350 µL of buffer S1, blowing away the bacteria, and transferring to a 2ml centrifuge tube; adding 250 µL of buffer S2, inverting upside down for 8 times; adding 250 µL of buffer S3, mixing upside down for 6 times, and producing a precipitate; centrifuging at 12000 rpm for 10 minutes, obtaining the supernatant and passing through a column; centrifuging for 1 minute, discarding the waste liquid, adding 500 µL of W1, centrifuging for 1 minute, and discarding the waste liquid; adding 750 µL of W2, centrifuging, and discarding the supernatant; adding 500 µL of W2, centrifuging, and discarding the supernatant; idling for 1 minute; adding 50 µL of eluent, settling for 2 minutes, and centrifuging. After concentration detection, 10 µL of the plasmid was taken for sequencing, and the positive plasmid was stored at -20°C. The sequencing results show that the obtained plasmid is capable of encoding the variant capsid protein VP1.

Further experimental results show that the prepared plasmid is capable of expressing the variant capsid protein VP1. The polynucleotide coding sequence of AAV-ie capsid VP1 is shown in SEQ ID NO. 5. The complete sequence of the constructed Rep-Cap plasmid is shown in SEQ ID NO. 6

### 2. Packaging and purification of AAV variant (named AAV-ie) virus

The obtained Rep-Cap plasmid, a genomic plasmid pAAV-CAG-mNeonGreen (the complete sequence of the plasmid is shown in SEQ ID NO. 11) expressing a green fluorescent protein mNeonGreen (GenBank: LC279210.1), and a pHelper plasmid (the complete sequence of the plasmid is shown in SEQ ID NO. 12) were co-transfected into HEK-In 293T cells at an appropriate amount. The AAV virus was purified by ultra-high-speed centrifugation with iododiol gradient. The virus titer was measured at a suitable concentration of 1E+12-1E+13 GC/mL, and placed at -80 °C for use.

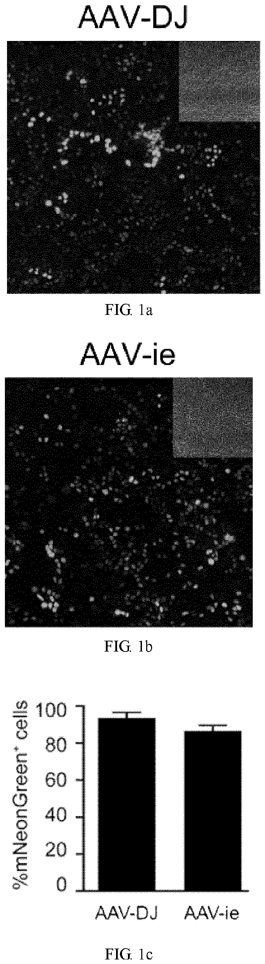

AAV viruses (AAV-ie and AAV-DJ, respectively) produced by the capsid protein variant and parental capsid protein were added to HEK 293T cells which were cultured with DMEM + 10% fetal bovine serum. The MOI value of the added virus is 1000. After 48 hours, the expression of green fluorescent protein mNeonGreen was observed by a fluorescence microscope, as shown in FIGs. 1a-1c. FIGs. 1a and 1b show the signal of the green fluorescent protein, and the upper right corner shows the cells under bright field natural light.

The results show that AAV variants and their parental AAV infect HEK 293T cells at a similar ratio.

### Embodiment 2 Infections of mouse cochlear tissue in vitro by AAV variants

Quickly removing the cochlea of P3 wild-type C57 mice (Shanghai Lingchang Biological Technology Co., Ltd.) on the ice, attaching the cochlea to a slide coated with cell-tak, and placing the slide in 98% DMEM + 1% N₂ + 1% Amp (5 µg/mL) medium for stabilizing for 12 h, and adding 1% FBS and 2×10¹⁰GC AAV to culture for 48-60h. Identifying the cultured samples by immunostaining. The samples were immersed in 4% Paraformaldehyde (PFA)for fixation, and immersed in phosphate buffer saline (PBS) containing 10% donkey serum and 0.3% Triton X-100. After incubating at room temperature for 1 hour, adding the antibodies of Myo7a (myosin 7a) and Sox2 protein and the corresponding secondary antibodies. The samples were mounted with an anti-fluorescence quenching agent mounting medium, and observed with confocal microscopy.

When shooting images by the confocal method, the laser power setting for shooting variant-infected samples was selected as the standard. All visible green fluorescent protein mNeonGreen signals were captured with the same laser settings.

In terms of data processing, by calculating the numbers of mNeonGreen-positive supporting cells and hair cells, the percentage of mNeonGreen on the cochlea was manually quantified. The cells with Myo7a-positive are hair cells, and the cells with Sox2 protein-positive are supporting cells.

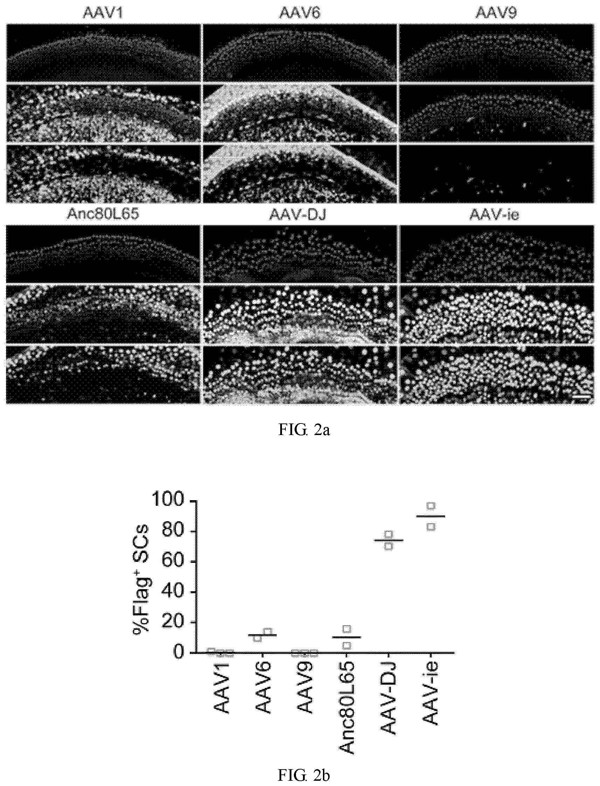

As shown in FIGs. 2a-2b, magenta represents the supporting cells, green represents the green fluorescent protein mNeonGreen expressed in the AAV-introduced cells, and FIG. 2b shows the statistical data of FIG. 2a. The results show that the AAV variant AAV-ie can efficiently infect the supporting cells of the in-vitro cultured mouse cochlear tissue. Compared with other AAV and the parental AAV, the proportion of Sox2-positive cells infected by AAV-ie is higher.

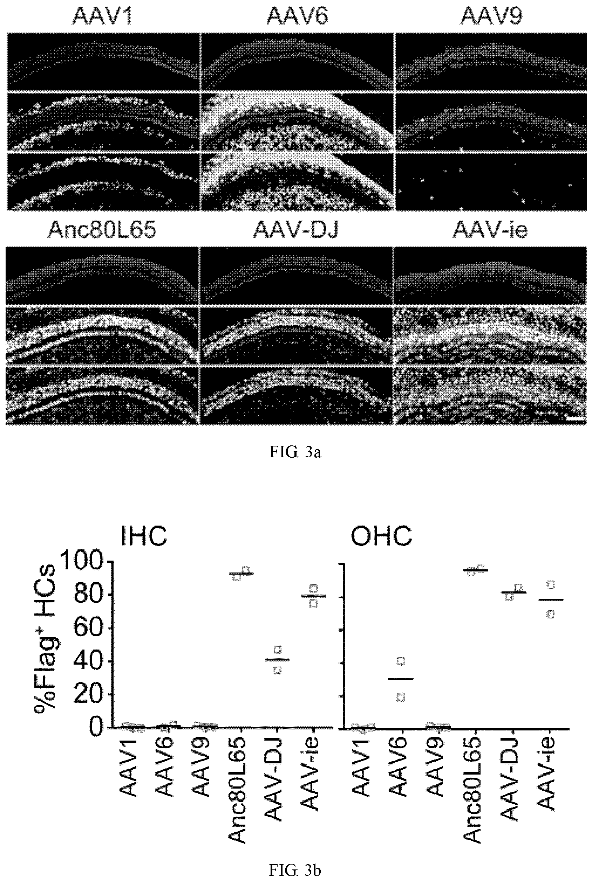

As shown in FIGs. 3a-3b, magenta represents the supporting cells, green represents the green fluorescent protein mNeonGreen expressed in the AAV-introduced cells, and FIG. 3b shows the statistical data of FIG. 3a. OHC represents outer hair cells, and IHC represents inner hair cells. The results show that the AAV variant can efficiently infect the hair cells of the in-vitro cultured mouse cochlear tissue. Compared with AAV1, AAV6, AAV9, Anc80L65 (the construction method is the same as that in Embodiment 1, the only difference is the Rep-Cap plasmid used, and the plasmid sequence is shown in SEQ ID NO. 7-10) and AAV-DJ, the variant AAV-ie has a higher infection rate to Myo7a-positive hair cells.

The above results indicate that the AAV variant AAV-ie can efficiently infect Myo7a-positive hair cells and Sox2 protein-positive supporting cells.

### Embodiment 3 AAV variants can efficiently infect various tissue cells in mouse cochlea after in-vivo injection

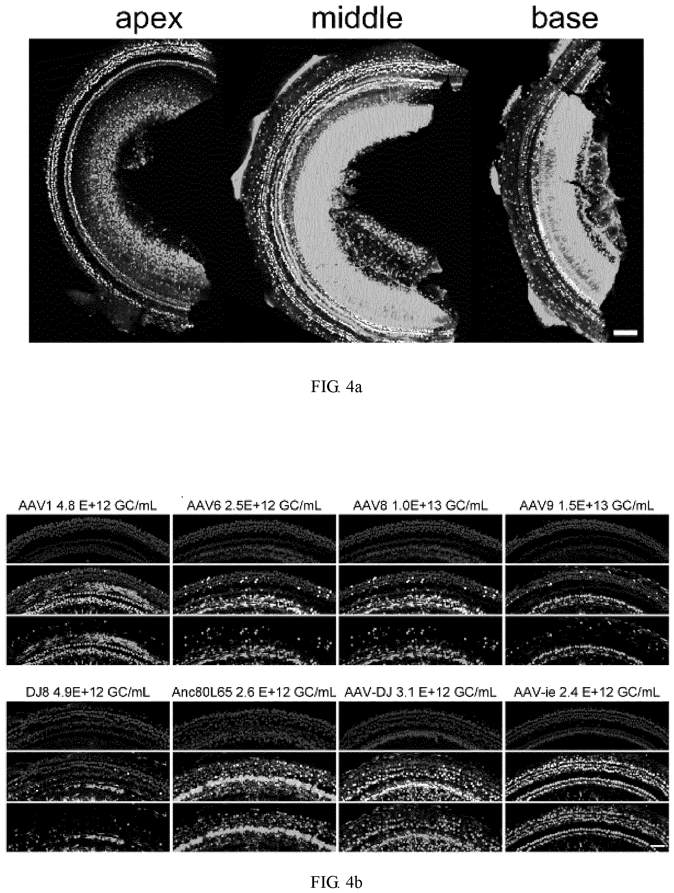

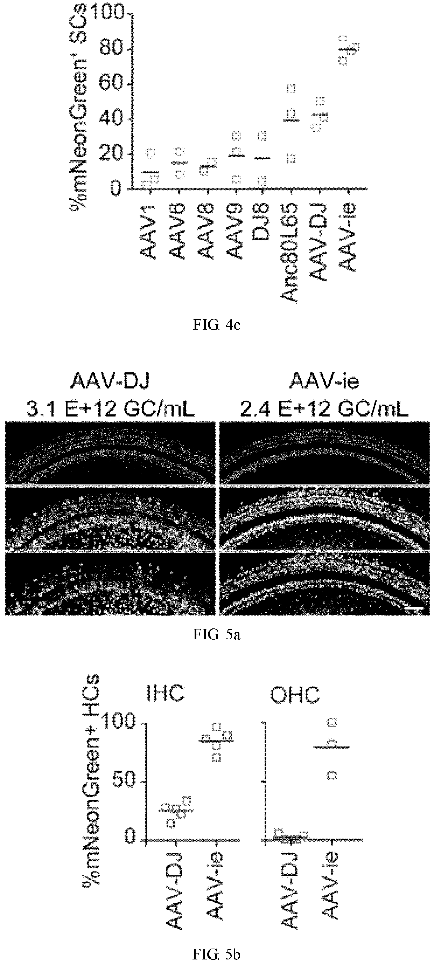

By using the cochlear round window injection technique, 1.5 µL of AAV variant viruses (the concentrations of the viruses are shown in FIG. 4) were injected into the cochlear perilymph. The specific steps are as follows: the newborn mice were anesthetized by low-temperature induced anesthesia method. P2-3 mice were placed in an ice bath for 2-3 minutes, and removed to an ice pad for subsequent surgical procedures. The operation was performed only in the left ear of each mouse, and the right ear served as a negative control. During the operation, an incision was made behind the left ear and the round window was exposed according to the relative positional relationship between the temporal bone and facial nerve. Avoid damage to the facial nerve during surgery. Next, using a micro-sampling system (Nanoliter 2000, WPI) to inject the AAV into the cochlea through the round window by a capillary glass electrode (diameter of 10 mm). The cochlea of a young mouse may hold 2 µL of AAV virus solution. The volume of the injected virus is 1-2 µL. After the operation, suturing the wound, applying the painkiller and the anti-inflammatory drug.

The mouse strain used was C57/B6. 10 days after the injection, peeling out the cochlea. Identifying the samples by immunostaining. The samples were immersed in 4% PFA for fixation, then immersed in PBS containing 10% donkey serum and 0.3% Triton X-100. After incubating at room temperature for 1 hour, adding the antibodies of Myo7a, Sox2, NeurN and the corresponding secondary antibodies. The samples were mounted with an anti-fluorescence quenching agent mounting medium, and observed with confocal microscopy.

As shown in FIGs. 4a-4c, magenta represents the Sox2-positive supporting cells, green represents the green fluorescent protein mNeonGreen expressed in the AAV-introduced cells. FIG. 4c represents the counting statistics of FIG. 4b. The result shows: 1. FIG. 4a shows the expression of green fluorescence mNeonGreen and the staining results of the supporting cell marker protein Sox2 represented by magenta in three areas of the cochlea tissue photographed in a large field of view: apex, middle and base. It can be found that AAV-ie can efficiently introduce fluorescent protein into various cells of the cochlea; 2. FIGs.4b and 4c show that the AAV variant can efficiently infect supporting cells in the mouse cochlear tissue in vivo. Compared with AAV1, AAV6, AAV8, AAV9, DJ8, Anc80L65 and AAV-DJ, the variant AAV-ie has a higher infection rate to Sox2-positive supporting cells.

As shown in FIGs. 5a-5b, magenta represents the Myo7a-positive hair cells, green represents the green fluorescent protein mNeonGreen expressed in the AAV-introduced cells. IHC represents inner hair cells, OHC represents outer hair cells. FIG. 5b represents the counting statistics of FIG. 5a. The results show that the AAV variant AAV-ie can efficiently infect the hair cells of the mouse cochlear tissue in vivo. Compared with the parental AAV, the variant AAV-ie has a higher infection rate to Myo7a-positive hair cells.

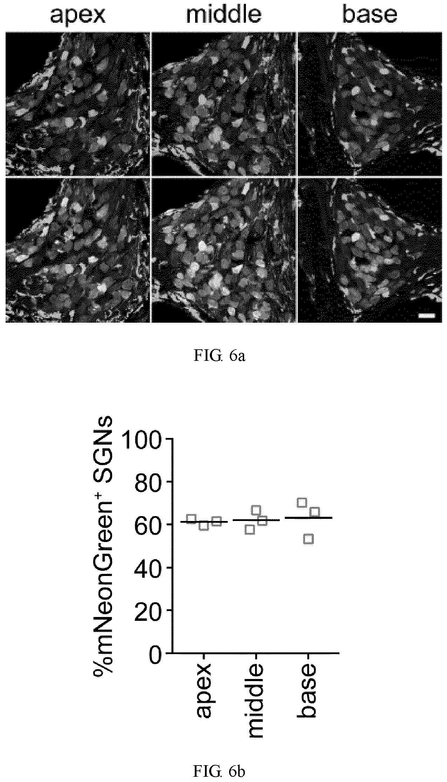

As shown in FIGs. 6a-6b, magenta represents the NeurN-positive spiral ganglion neurons, green represents the green fluorescent protein mNeonGreen expressed in the AAV-introduced cells. FIG. 6b represents the counting statistics of FIG. 6a. The results showed that in three areas of the cochlea (apex, middle and base), the AAV variant AAV-ie can efficiently infect the spiral ganglion neurons of the in-vitro cultured mouse cochlear tissue.

The above results indicate that the AAV variant using round window injection method can efficiently infect Myo7a-positive hair cells, Sox2-positive supporting cells and NeuroN-positive spiral ganglion neurons.

### Embodiment 4 In-vivo injection of Atoh1-carrying AAV-ie into the cochlea causes the regeneration of a large number of hair cells

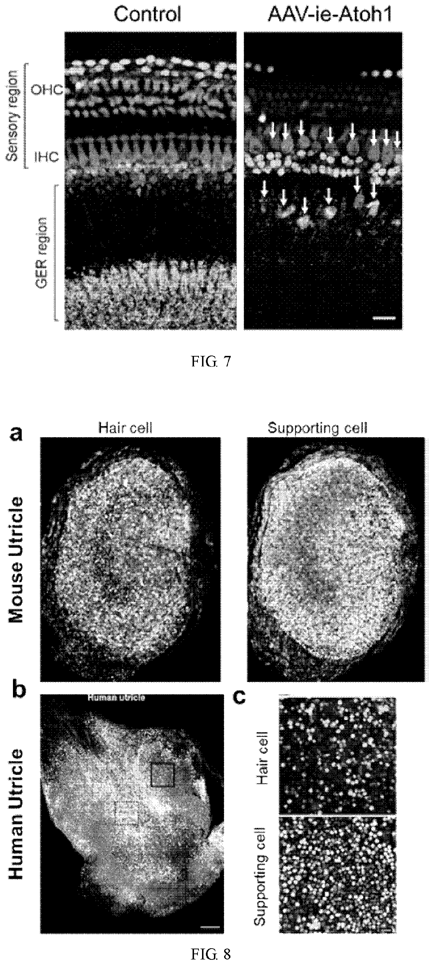

By using the cochlear round window injection technique, 1.5 µL of AAV-ie-Atoh1 viruses (virus concentration of 5E+12GC/mL) (Refer to embodiment 1 for construction method, the plasmid used was replaced by a plasmid expressing Atohl gene (NCBI Reference Sequence: NM_007500.5)) carrying mouse Atohl gene (NCBI Reference Sequence: NM_007500.5) were injected into the cochlear perilymph. For the specific method, please refer to Embodiment 3. The mouse strain used was C57/B6. The mice used were young mice 2-3 days after birth. 10 days after the injection, peeling out the cochlea. Identifying the samples by immunostaining. The samples were immersed in 4% PFA for fixation, and then immersed in PBS containing 10% donkey serum and 0.3% Triton X-100. After incubating at room temperature for 1 hour, adding the antibodies of Myo7a, Sox2 and the corresponding secondary antibodies. The samples were mounted with an anti-fluorescence quenching agent mounting medium, and observed with confocal microscopy. The results are shown in FIG. 7, magenta represents the immunostaining of the hair cell marker gene Myo7a, green represents the immunostaining of the supporting cell marker gene Sox2, and white arrows represent ectopically regenerated hair cells.

The above results show that AAV-ie-Atoh1 virus can significantly regenerate hair cells in the sensory region and GER region after introducing Atohl gene into the supporting cells.

### Embodiment 5 AAV-ie can efficiently infect mouse and human utricle cells

The utricle is an organ in the inner ear that senses gravity and maintains balance. By using the cochlear round window injection technique, 1.5 µL of AAV-ie viruses (concentration of the virus is 6E+12 GC/mL) were injected into the cochlear perilymph. For the specific method, please refer to Embodiment 3. The mouse strain used was C57/B6. The mice used were young mice 2-3 days after birth. 10 days after the injection, peeling out the utricle. Identifying the samples by immunostaining. The samples were immersed in 4% PFA for fixation, and then immersed in PBS containing 10% donkey serum and 0.3% Triton X-100. After incubating at room temperature for 1 hour, adding the antibodies of Myo7a, Sox2 and the corresponding secondary antibodies. The samples were mounted with an anti-fluorescence quenching agent mounting medium, and observed with confocal microscopy. As shown in FIG. 8a, magenta represents the hair cell marker protein Myo7a and the supporting cell marker protein Sox2, respectively, green represents the green fluorescent protein mNeonGreen delivered by AAV-ie. It can be found that AAV-ie can efficiently infect the hair cells and supporting cells of the utricle of the mice.

Taking the utricle of human, and infecting with 5 × 10¹⁰ GCs of AAV-ie virus. After 7 days, the human samples were immersed in 4% PFA for fixation, and then immersed in PBS containing 10% donkey serum and 0.3% Triton X-100. After incubating at room temperature for 1 hour, adding the antibodies of Myo7a, Sox2 and the corresponding secondary antibodies. The samples were mounted with an anti-fluorescence quenching agent mounting medium, and observed with confocal microscopy. As shown in FIGs. 8b-8c, green represents the green fluorescent protein mNeonGreen delivered by AAV-ie. Red represents the hair cell marker protein Myo7a, and magenta represents the supporting cell marker protein Sox2. The result shows that AAV-ie is also capable of efficiently infect the hair cells and supporting cells of the utricle of human.

The above embodiments are intended to illustrate the disclosed embodiments of the present disclosure and are not understood as restrictions on the present disclosure. In addition, various modifications of the present disclosure, as well as variations of the methods and compositions of the disclosure, will be apparent to those skilled in the art without departing from the scope of the disclosure. While the disclosure has been described in detail in connection with various specific preferred embodiments thereof, however, it should be understood that the present invention should not be limited to these specific embodiments. In fact, various modifications to the disclosure as apparent to those skilled in the art are intended to be included within the scope of the disclosure.

## Claims

1. A capsid protein VP1 of a variant adeno-associated virus AAV-ie, wherein an amino acid fragment is inserted between N589 and R590 of a capsid protein VP1 of a wild-type AAV-DJ, and an amino acid sequence of the amino acid fragment is shown in SEQ ID NO.1.

2. The capsid protein VP1 of the variant adeno-associated virus AAV-ie according to claim 1, wherein an amino acid sequence of the capsid protein VP1 of the variant adeno-associated virus AAV-ie is shown in SEQ ID NO. 4.

3. An isolated nucleic acid, comprising a nucleotide sequence encoding the capsid protein VP1 of the variant adeno-associated virus AAV-ie according to claim 1.

4. A construct, comprising the isolated nucleic acid according to claim 3.

5. A host cell, comprising the construct according to claim 4 or incorporating an exogenous isolated nucleic acid according to claim 3 in a genome.

6. A variant adeno-associated virus AAV-ie, comprising the capsid protein VP1 of the variant adeno-associated virus AAV-ie according to any one of claims 1-2.

7. The variant adeno-associated virus AAV-ie according to claim 6, wherein the variant adeno-associated virus AAV-ie further comprises a heterologous nucleotide sequence encoding a target product.

8. The variant adeno-associated virus AAV-ie according to claim 6, wherein the target product is a nucleic acid or a protein, and the nucleic acid is preferably selected from small guide RNA or interfering RNA.

9. A pharmaceutical composition, comprising the variant adeno-associated virus AAV-ie according to any one of claims 6-8 and pharmaceutically acceptable excipients.

10. Use of the variant adeno-associated virus AAV-ie according to any one of claims 6-8 for delivering a target product to hair cells and/or supporting cells of an individual.

11. The use according to claim 10, wherein the target product is a nucleic acid or a protein, and the nucleic acid is preferably selected from small guide RNA or interfering RNA.

12. Use of the variant adeno-associated virus AAV-ie according to any one of claims 6-8 in preparation of drugs for treatment of a hearing impairment disease caused by cochlear injury in an individual.

13. The use according to claim 12, wherein the hearing impairment disease is a disease related to genetic defects, environmental damage or aging.

14. The use according to claim 12, wherein the hearing impairment disease is a disease related to cell damage.
